# EUROPEAN PATENT SPECIFICATION

(11) **EP 2 970 118 B1**
(45) Date of publication and mention of the grant of the patent: **12.09.2018**
(21) Application number: 14779508.2
(22) Date of filing: 06.03.2014
(51) Int. Cl.: C07D 209/04, A61K 31/404, A61P 25/28

(54) **COMPOUNDS FOR THE TREATMENT OF NEUROLOGICAL DISORDERS**
VERBINDUNGEN ZUR BEHANDLUNG NEUROLOGISCHER ERKRANKUNGEN
COMPOSÉS POUR LE TRAITEMENT DE TROUBLES NEUROLOGIQUES

(30) Priority: 13.03.2013 US 201313801348; 08.08.2013 GB 201314198
(43) Date of publication of application: 20.01.2016
(73) Proprietor: Manfredi, John, Salt Lake City, UT 84108 (US)
(72) Inventor: Manfredi, John, Salt Lake City, UT 84108 (US)
(74) Representative: Bradley, Adrian
(86) International application number: PCT/US2014/021331
(87) International publication number: WO 2014/164222

(56) References cited:
- WO-A2-2011/119777
- US-A1- 2009 099 179
- US-A1- 2013 261 118
- US-B2- 7 678 823
- US-B2- 8 203 004

## Description

### TECHNICAL FIELD OF THE INVENTION

The present disclosure provides compounds, compositions and methods of synthesizing compounds that are effective against neurodegenerative disorders, including their action as neurotrophic and axonotrophic agents. The present disclosure provides methods for the therapeutic treatment of neurodegenerative disorders, and methods of prophylaxis against neurodegenerative disorders. The present disclosure further provides pharmaceutical compositions for use in the disclosed methods of the invention.

### BRIEF DESCRIPTION OF THE DRAWINGS

FIG. 1 illustrates the pharmacologic suppression of the locomotion defect of kinesin-deficient *Drosophila* larvae in the presence of exemplary compounds.
FIG. 2 shows the reduction of neuronal aggregates in segmental nerves of *Drosophila* larvae by an exemplary compound.
FIG. 3 illustrates the stimulation of neurite outgrowth in isolated rat spinal motor neurons in the presence of exemplary compounds.
FIG. 4 illustrates the stimulatory effect of exemplary compounds on axonal outgrowth of motor neurons in cultured sections of rat spinal cord.
FIG. 5 illustrates the rescue of motor axon development in Smn-deficient zebrafish embryos by an exemplary compound.
FIG. 6 illustrates the rescue of motor axon development in Smn-deficient zebrafish embryos by exemplary compounds.

### SUMMARY OF THE INVENTION

The claimed subject-matter is defined in the appended claims. The present disclosure provides compounds, compositions and methods of synthesizing compounds that are effective against neurodegenerative diseases and disorders, including their action as neurotrophic and axonotrophic agents.

In an aspect, the invention provides compounds of Formula I, pharmaceutically acceptable salts thereof, and pharmaceutical compositions containing such compounds. for use in a method of treating a neurodegenerative disease or disorder associated with compromised axonal function selected from those recited in claim 1.

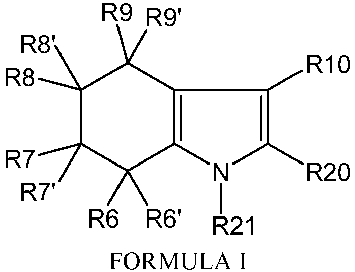

In a further aspect, the invention provides the claimed compounds for use in a method for prophylaxis against, or delaying the onset of, a neurodegenerative disorder, by identifying a patient in need of or desiring such treatment, and administering to the patient a prophylactically effective amount of a pharmaceutical composition having one or more compounds of Formula I. The pharmaceutical composition for use in the invention may be formulated with one or more pharmaceutically acceptable excipients, salts, or carriers. The pharmaceutical composition for use in the invention may be delivered orally, such as in a tablet or capsule dosage form. Patients having a predisposition to a neurodegenerative disorder or suspected of needing prophylaxis or a delay of the onset of the disease (or symptoms thereof), can be identified by any method known to the skilled artisan for diagnosis such neurodegenerative disorders.

In an aspect, the invention provides the claimed compounds for use in a method of preventing the onset of a neurodegenerative disorder comprising administering to a patient in need of or desiring such treatment, a pharmaceutical composition having one or more compounds of Formula I.

In further aspects, the invention provides compounds of Formula I for use in treating, delaying and/or preventing disorders associated with axonal transport defects. In some aspects, the invention provides for the use of compounds of Formula I for treating and/or preventing disorders characterized by, or associated with, a defect in vesicular transport, including axonal transport.

In another embodiment, the invention provides a method of treating a disorder associated with a defect in axonal transport, by identifying a patient in need of such treatment, and administering to the patient a therapeutically effective amount of a pharmaceutical composition having one or more compounds of Formula I. The pharmaceutical composition for use in the invention may be formulated with one or more pharmaceutically acceptable excipients, salts, or carriers.

In yet another embodiment, the invention provides a method for prophylaxis against a disorder associated with a defect in axonal transport, by identifying a patient in need of or desiring such treatment, and administering to the patient a prophylactically effective amount of a pharmaceutical composition having one or more compounds of Formula I. Compounds for use in this embodiment of the invention include those in Table 1. Administration of a compound of Formula I may delay the onset of the disorder or slow the rate of onset of symptoms of the disorder.

In an aspect, the invention provides a method of treating a disease chosen from amyotrophic lateral sclerosis (ALS), Charcot-Marie-Tooth Disease 2 (CMT2), spinal muscular atrophy (SMA), hereditary sensory motor neuropathy, hereditary spastic paraplegia, multiple sclerosis, comprising administering to a patient in need of such treatment, a pharmaceutical composition having one or more compounds of Formula I.

The foregoing and other advantages and features of the invention, and the manner in which the same are accomplished, will become more readily apparent upon consideration of the following detailed description of the invention taken in conjunction with the accompanying examples, which illustrate preferred and exemplary embodiments.

### DETAILED DESCRIPTION OF THE INVENTION

The present disclosure provides compounds, compositions and methods of synthesizing compounds that are effective against neurodegenerative diseases and disorders, including their action as neurotrophic and axonotrophic agents, as disclosed herein. Exemplary neurodegenerative diseases and disorders include spinal muscular atrophy (SMA), Charcot-Marie-Tooth Type 2 disease (CMT2), hereditary spastic paraplegia (HSP), hereditary sensory motor neuropathy, amyotrophic lateral sclerosis (ALS), Huntington's disease, Parkinson's disease, spinocerebellar ataxia 3 (SCA3), spinocerebellar ataxia 6 (SCA6), spinocerebellar ataxia 5 (SCA5), spinobulbar muscular atrophy (SBMA), dystonia musculorum, frontotemporal dementia with parkinsonism linked to chromosome 17 (FTDP-17), multiple sclerosis (MS), frontotemporal lobar degeneration (FTLD) / frontotemporal dementia (FTD), spinal cord injury (SCI), and Alzheimer's disease. Also disclosed are methods of using the described compounds and compositions to treat such neurodegenerative diseases and disorders.

The disclosure herein specifically addresses the ability of the claimed neurotrophic and axonotrophic agents to correct axonal dysfunction in motor neurons, which is critical in a number of diseases and disorders affecting such neurons, including the motor neuropathy SMA. Methods of treating various diseases and disorders involving compromised axonal function are possible, as the "downstream" dysfunction may be alleviated by treatment with the exemplary compounds of the invention to correct any of a number of "upstream" defects that lead to the compromised axonal function.

Proximal spinal muscular atrophy (SMA), a common inherited motor neuropathy for which there is no effective treatment, results from inadequate levels of the ubiquitously expressed survival motor neuron protein (SMN) (Monani, Neuron 2005, 48(6):885-896). Given the expression of SMN throughout the body, it is paradoxical that its deficiency preferentially affects motor neurons in the anterior horn of the spinal cord (Burghes et al, Nat Rev Neurosci 2009, 10(8):597-609). This suggests that some distinguishing and essential features of spinal motor neurons are particularly vulnerable to SMN deficiency. One such feature is the neuromuscular junction - a highly specialized structure that develops where the motor axon terminates on muscle and that is dependent on the proper function of the distal motor axon and its terminus (Murray et al, Neuropathol Appl Neurobiol 2010, 36(2):133-156). It is possible, then, that a primary consequence of SMN deficiency is dysfunction of distal motor axons and their termini. Indeed, SMN deficiency is reported to disrupt processing of pre-mRNAs encoding subunits of kinesin and dynein (*viz.,* Kif17, Klc4, and Dync1h1; Zhang et al, Cell 2008, 133(4):585-600), which drive transport of diverse cargoes in axons (Hirokawa Science 1998, 279(5350):519-526). The function and viability of distal axons and termini of spinal motor neurons, given their exceptional lengths, will be particularly dependent on the activities of these motors.

Another transcript that suffers disrupted processing as a result of SMN deficiency encodes Stasimon, whose resulting deficiency alters neurotransmitter release at motor axon termini (Lotti et al, Cell 2012, 151(2):440-454). Inadequate SMN levels also compromise the formation of messenger ribonucleoproteins (mRNPs), which are complexes that that regulate mRNA transport, stability, and local translation in axons. The consequently reduced levels of the encoded proteins in axons and their growth cones can severely affect the function of axon termini (Akten et al, Proc Natl Acad Sci USA 2011, 108(25):10337-10342; Fallini et al, J Neurosci 2011, 31(10):3914-3925; Rossoll et al, J Cell Biol 2003, 163(4):801-812). Yet other mechanisms by which reduced SMN levels can adversely affect axonal function involve the interaction of SMN with both plastin 3, which promotes axonogenesis via its effects on actin (Delanote et al, Acta Pharmacol Sin 2005, 26(7):769-779; Oprea et al, Science 2008, 320(5875):524-527), and profilin II, which influences growth cone motility via its effects on actin and ROCK (Bernard Int J Biochem Cell Biol 2007, 39(6):1071-1076; Da Silva et al, J Cell Biol 2003, 162(7):1267-1279; Tang Neurochem Int 2003, 42(3):189-203; Gutsche-Perelroizen et al, J Biol Chem 1999, 274(10):6234-6243; Witke et al, EMBO J 1998, 17(4):967-976; Bito et al, Neuron 2000, 26(2):431-441; Bowerman et al, Mol Cell Neurosci 2009, 42(1):66-74; Bowerman et al, J Mol Neurosci 2007, 32(2):120-131; Kim et al, J Neurobiol 2001, 47(1):26-38; Wills et al, Neuron 1999, 22(2):291-299). Through all of these disparate mechanisms, low SMN levels can compromise the function of the distal motor axon and its terminus and thereby contribute to SMA pathology. Accordingly, agents that enhance the growth, development, and performance of motor axons *(i.e.,* axonotrophic agents) may be therapeutic for SMA and other neurological diseases involving compromised axonal function.

Such axonotrophic agents may be found among compounds that affect the processing of amyloid precursor protein (APP), as there is an association between the processing of APP and intracellular membrane trafficking. For example, kinesin deficiency in mice is reported to alter γ-secretase-mediated processing of APP (Stokin et al, Science 2005, 307(5713):1282-1288). Specifically, deletion of one copy of the kinesin light chain increased the levels of the APP-derived peptides Aβ42 and Aβ40 in brains of mice that express APP mutants associated with Alzheimer's disease. Thus, kinesin function and metabolism of Aβ peptides may be mechanistically coupled. Given the importance of kinesin for the growth, development, and function of motor axons (Hurd et al, Genetics 1996, 144(3):1075-1085), agents that modulate Aβ metabolism may therefore have axonotrophic activities.

### Methods of Prevention and Treatment

While not wishing to be bound by theory, it is believed that the compounds of Formula I act *in vivo* to treat and/or prevent certain pathologies by correcting axonal dysfunction by specifically modulating a biochemical pathway associated with a vesicular transport pathway (e.g., axonal transport). Such diseases include, but are not limited to, amyotrophic lateral sclerosis (ALS), Charcot-Marie-Tooth Disease 2 (CMT2), spinal muscular atrophy (SMA), Parkinson's Disease (PD), hereditary sensory motor neuropathy, optic neuropathies (e.g., Leber's hereditary optic neuropathy [LHON] and Cuban epidemic of optic neuropathy [CEON]), Niemann-Pick type C disease (NPC), Down syndrome, Dementia with Lewy Bodies (DLB), Parkinson's disease, tauopathies (e.g., progressive supranuclear palsy, corticobasal degeneration, Pick's disease, argyrophilic grain disease, and frontotemporal dementia and parkinsonism linked to chromosome 17 [FTDP-17]), miscellaneous motor neuron disorders (e.g., primary lateral sclerosis [PLS]), hereditary spastic paraplegia (HSP), multiple sclerosis (MS), Guillain-Barré syndrome, traumatic brain injury, spinal cord injury, and polyQ diseases (e.g., Huntington disease, spinobulbar muscular atrophy, dentatorubral-pallidoluysian atrophy, Kennedy's disease (also called spinobulbar muscular atrophy [SBMA]), spinocerebellar ataxia 1, spinocerebellar ataxia 2, spinocerebellar ataxia 3, spinocerebellar ataxia 6, spinocerebellar ataxia 7, and spinocerebellar ataxia 17).

For example, the compounds of Formula I may be useful in the treatment of diseases including Charcot-Marie-Tooth Disease 2 (CMT2), spinal muscular atrophy (SMA), hereditary sensory motor neuropathy, hereditary spastic paraplegia (HSP), and multiple sclerosis (MS), all of which involve compromised axonal function.

The following section provides a brief description of disorders associated with a defect in vesicular transport.

PolyQ disease. The expansion of CAG repeats encoding glutamine is known to cause several late-onset progressive neurodegenerative disorders: Huntington disease, spinobulbar muscular atrophy, dentatorubral-pallidoluysian atrophy, Kennedy's disease (also called spinobulbar muscular atrophy [SBMA]), spinocerebellar ataxia 1, spinocerebellar ataxia 2, spinocerebellar ataxia 3, spinocerebellar ataxia 6, spinocerebellar ataxia 7, and spinocerebellar ataxia 17. These polyQ disorders commonly exhibit defects in axonal transport (Neuron. 40:1, 2003; Neuron 40:25, 2003; Neuron 40:41, 2003). Indeed, evidence suggests that perturbations in transport pathways are an early event in polyQ disease (Arch Neurol. 62:46, 2005).

Traumatic brain and spinal cord injury. Traumatic brain injury (TBI) is marked by rapid and long-term accumulation of proteins, including beta-amyloid precursor protein. TBI is also an epigenetic risk factor for developing neurodegenerative disorders, such as Alzheimer's disease and Parkinson's disease (Neuromolecular Med. 4:59, 2003).

Hereditary spastic paraplegia and spinal muscular atrophy. These motor neuron diseases exhibit clear cytoskeletal abnormalities that suggest the involvement of axonal transport in the pathogenesis of the diseases (Trends Neurosci. 25:532, 2002).

Multiple sclerosis. Inflammation is the cause of much neural damage in multiple sclerosis, resulting in disruption of axonal transport (Curr Opin Neurol. 16:267, 2003). These observations admit the possibility that the neurodegeneration experienced by MS patients may be attenuated by agents that enhance axonal transport. In a similar vein, diseases such as Guillain-Barré syndrome, an inflammatory disorder of the peripheral nerves, may be amenable to therapeutic intervention with agents that enhance axonal transport.

Miscellaneous motor neuron disorders. Primary lateral sclerosis (PLS) is a rare degenerative disorder of the upper motor neuron, whose classification is controversial (J Neurol Sci. 170:5, 1999). In fact, a recent study has concluded that PLS is not a discrete nosological entity but represents one end of a continuous spectrum of motor neuron disease (Brain 124:1989, 2001). A therapeutic that successfully treats one motor neuron dysfunction is therefore a candidate for treatment of other motor neuron disorders.

Tauopathies. Aberrant functions of the microtubule-associated proteins collectively called tau can lead to neurodegenerative disorders like progressive supranuclear palsy, corticobasal degeneration, Pick's disease, argyrophilic grain disease, and frontotemporal dementia and parkinsonism linked to chromosome 17 (FTDP-17) (Biochim Biophys Acta. 1739:240, 2005; Brain Res Brain Res Rev. 33:95, 2000). One feature of tauopathies is the disruption of axonal transport that accompanies them.

Dementia with Lewy Bodies. Dementia with Lewy Bodies (DLB) is characterized by the presence of cytoplasmic inclusions of alpha-synuclein in the cerebral cortex and in the nuclei of the brain stem (Arch Gerontol Geriatr 39:1, 2004). Protein aggregates, whether they are aggregates of tau, Aβ, prions or other proteins, apparently disrupt vesicle transport. A therapy that treats dysfunctional vesicle transport is a candidate regimen for treatment of DLB.

Down syndrome. Nearly all individuals with Down syndrome develop amyloid plaques and the attendant neuropathologic lesions by the age of 45 (Arch Neurol 46:849, 1989). Since accumulation of amyloidogenic Aβ peptides in brain is sensitive to the function of kinesin (Stokin 2005), a motor protein that is critical to axonal function, compounds that affect kinesin function may, by influencing the development of amyloid plaques, moderate or delay the onset of the dementia of Down syndrome.

Niemann-Pick type C disease (NPC). The primary lesion of NPC appears to be impaired cholesterol trafficking and excessive glycosphingolipid storage. One consequence of this impairment is abnormal vesicle trafficking in neural tissue, which likely contributes to the neurodegeneration characteristic of the disease (Neurobiol Aging 26:373, 2005). A recent study indicates that the abnormal vesicle trafficking contributes to increased deposition of Aβ42 in brain tissue of NPC patients (Am J Pathol. 164:975, 2004), which suggests that Aβ peptides may participate in the neurodegeneration.

Optic neuropathies. Histological evidence suggests impaired axonal transport of mitochondria in Leber's hereditary optic neuropathy (LHON) and in Cuban epidemic of optic neuropathy (CEON). Since mitochondria are transported along microtubules by mechanisms similar to microtubule-directed transport of vesicles, agents that enhance transport of vesicles are expected to enhance axonal transport of mitochondria.

Parkinson's disease. Impaired axonal transport has been documented in brains affected by Parkinson's disease and shown to be prominently involved in the pathogenesis of the disease (Pienaar et al, Mol Neurobiol 2012, 45(1):126-43; De Vos et al, Annu Rev Neurosci 2008, 31:151-73; Acta. Neuropathol.(Berl) 98:157-164, 1999).

Amyotrophic lateral sclerosis. (J. Neurol. Sci. 63:241-250, 1984; Acta. Neuropathol. (Berl) 94:294-299, 1997). Despite the myriad causes of ALS, a common feature of the disease is impaired axonal transport of both vesicles (Palmisano et al, BMC Neurosci 2011, 12:24; Laird et al, J Neurosci 2008, 28:1997-2005) and mitochondria (Mórotz et al, Hum Mol Genet 2012, 21:1979-88; Magrané and Manfredi, Antioxid Redox Signal 2009, 11:1615-26).

In summary, axonal function is compromised in all of these diseases. Furthermore, an extensive body of research supports the concept that agents that rescue more normal axonal function would mitigate the pathology of these diseases.

The skilled artisan readily recognizes that the compounds and pharmaceutical compositions can be used to treat diseases other than those listed herein, which involve some type of axonal dysfunction.

### Compounds

In general, the invention relates to compounds of Formula I, pharmaceutically acceptable salts thereof, and pharmaceutical compositions containing the compounds and salts for use in the treatment and prophylaxis of neurodegenerative disorders recited in claim 1.

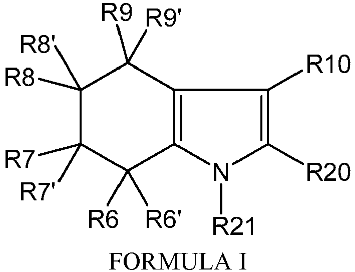

In Formula I, the bonds from the saturated ring to the substituents R6, R6', R7, R7', R8, R8', R9 and R9' are not intended to show any chirality unless otherwise noted, although they each may independently be chiral. All chiral conformations and combinations thereof are included in the compounds of Formula I. For example, the R-group pair R6 and R6' are both attached to the same carbon in the saturated ring, but no chirality is indicated. When different substituents are recited for the R-group pairs, there is no chirality assumed or intended by the order of recitation, although all conformations are included. Some of the compounds of Formula I for use in the invention may exist as single stereoisomers (*i.e*., essentially free of other stereoisomers), racemates, and/or mixtures of enantiomers and/or diastereomers. All such single stereoisomers, racemates and mixtures thereof are intended to be within the scope of the present invention. Furthermore, some of the compounds for use in the invention can exist as *cis* and *trans* geometric isomers, and all such isomers and mixtures thereof are intended to be within the scope of the present invention.

In the compounds of Formula I, the groups labeled R6-R10 are independently selected from at least one of hydro, hydroxyl, halo, alkyl, alkoxy, haloalkyl, haloalkoxy,-N(C₁₋₃ alkyl)₂, -NH(C₁₋₃ alkyl), -C(=O)NH₂, -C(=O)NH(C₁₋₃ alkyl), -C(=O)N(C₁₋₃ alkyl)₂,-S(=O)₂(C₁₋₃alkyl), -S(=O)₂NH₂, -S(=O)₂N(C₁₋₃ alkyl)₂, -S(=O)₂NH(C₁₋₃ alkyl), -CHF₂, -OCF₃, -OCHF₂, -SCF₃, -CF₃, -CN, -NH₂, and -NO₂. For example, R6 and R6' may be hydro; R7 and R7' may independently be hydro, methyl, ethyl or trifluoromethyl; R8 and R8' may independently be hydro, methyl, tert-butyl, 1,1-dimethylpropyl, trifluoromethyl, cyclohexyl or methyl; R9 and R9' may independently be hydro or trifluoromethyl, and R10 may be hydro.

In certain embodiments, R6 and R6' are hydro, R7, R7', R8 and R8' are independently selected from at least one of hydro, alkyl and haloalkyl, R9 is alkyl or haloalkyl, R9' is hydro, and R10 is hydro. In further embodiments, R6, R6', R7, R7', R8, R9, R9' and R10 may be hydro and R8' is 1,1-dimethylpropyl or trifluoromethyl.

In the compounds of Formula I, the group labeled R20 is aryl or heteroaryl, each of which may be optionally substituted. For example, R20 may be phenyl, 3,4-dichlorophenyl, 4-morpholin-4-yl-phenyl, 2-benzofuran-2-yl, 4-trifluoromethylphenyl, 2,4-dimethoxyphenyl, 3,4-dimethoxyphenyl, or 3-chlorophenyl. In an embodiment, R20 is aryl or benzofuranyl. In an embodiment, R20 is phenyl or 3,4-dichlorophenyl.

In the compounds of Formula I, the group labeled R21 is heteroaryl, which optionally may be substituted, and which may be joined to the nitrogen atom via any appropriate position of the heteroaryl ring. For example, R21 may be furan-2-carboxylic acid, thiazol-4-yl acetic acid, picolinic acid, nicotinic acid, isonicotinic acid, pyrimidine-4-carboxylic acid, 1H-pyrrole-2-carboxylic acid, furan-2-carboxylic acid methyl ester, thiazole-4-carboxylic acid, or 4H-[1,2,4]triazol-3-yl. In an embodiment, R21 is heteroaryl substituted with a carboxy or a carboxyalkyl. In certain embodiments, R21 is furan-2-carboxylic acid or thiazol-4-yl acetic acid.

The compounds of Formula I include pharmaceutically-acceptable salts thereof.

In certain embodiments, when R9 is OH, R9' is hydro, and R7 and R7' are each methyl, R21 is not 3-pyridyl. Particularly, R9 can be OH, R9' can be hydro, and R7 and R7' can each be methyl in the compounds of Formula I, with the proviso that when R9 is OH, R9' is hydro, and R7 and R7' are each methyl, the compound of Formula I is not 6,6-dimethyl-2-phenyl-1-(pyridin-3-yl)-4,5,6,7-tetrahydro-1H-indol-4-ol.

In certain embodiments, when R6, R6', R7, R7', R8, R8', R9, R9' and R10 are all hydro and R20 is unsubstituted phenyl, R21 is not 3-pyridyl. Particularly, R6, R6', R7, R7', R8, R8', R9, R9' and R10 can all be hydro and R20 can be unsubstituted phenyl in the compounds of Formula I, with the proviso that when R6, R6', R7, R7', R8, R8', R9, R9' and R10 are all hydro and R20 is unsubstituted phenyl, the compound of Formula I is not 2-phenyl-1-(pyridin-3-yl)-4,5,6,7-tetrahydro-1H-indole.

In an embodiment, exemplary compounds include compounds having the structure of Formula I wherein R6, R6' and R10 are hydro, R7, R7', R8 and R8' are independently selected from at least one of hydro, alkyl and haloalkyl, R9 is alkyl or haloalkyl, R9' is hydro, R20 is aryl or benzofuranyl, and R21 is heteroaryl. In certain embodiments, exemplary compounds include compounds having the structure of Formula I wherein R6, R6', R7, R7', R8, R9, R9' and R10 are hydro, R8' is 1,1-dimethylpropyl or trifluoromethyl, R20 is phenyl or 3,4-dichlorophenyl, and R21 is furan-2-carboxylic acid or thiazol-4-yl acetic acid.

Exemplary compounds of Formula I and analogs thereof include the compounds shown in Table 1.

**Table 1. Exemplary compounds of Formula I and analogs thereof.**

| compound structure | compound name | compound abbrev. |
|---|---|---|
| 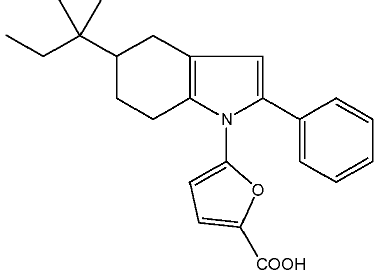 | 5-[5-(1,1-dimethyl-propyl)-2-phenyl-4,5,6,7-tetrahydro-indol-1-yl]-furan-2-carboxylic acid | A |
| 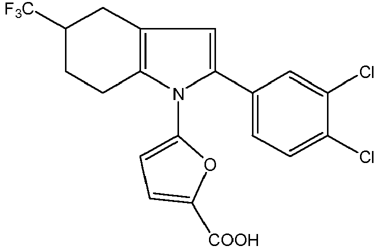 | 5-[2-(3,4-dichloro-phenyl)-5-trifluoromethyl-4,5,6,7-tetrahydro-indol-1-yl]-furan-2-carboxylic acid | B |
| 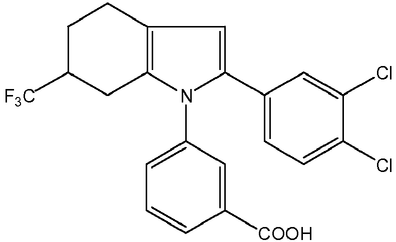 | 3-[2-(3,4-dichloro-phenyl)-6-trifluoromethyl-4,5,6,7-tetrahydro-indol-1-yl]-benzoic acid | C |
| 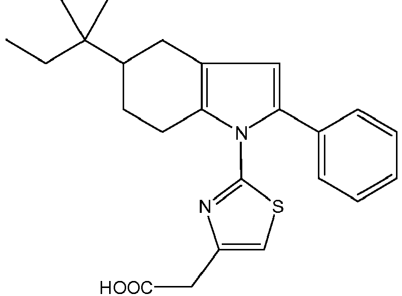 | {2-[5-(1,1-dimethylpropyl)-2-phenyl-4,5,6,7-tetrahydroindol-1-yl]-thiazol-4-yl} acetic acid | D |
| 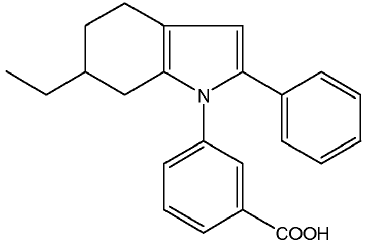 | 3-(6-ethyl-2-phenyl-4,5,6,7-tetrahydroindol-1-yl) benzoic acid | E |
| 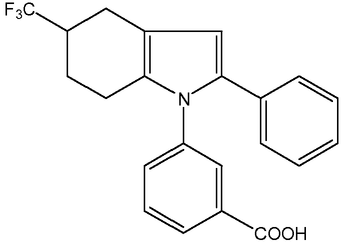 | 3-(2-phenyl-5-trifluoromethyl-4,5,6,7-tetrahydroindol-1-yl) benzoic acid | F |
| 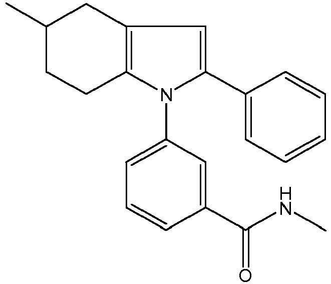 | N-methyl-3 -(5 -methyl-2-phenyl-4,5,6,7-tetrahydroindol-1-yl) benzamide | G |
| 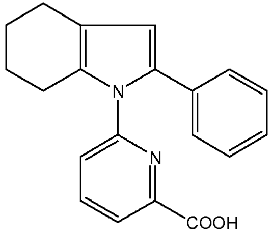 | 6-(2-phenyl-4,5,6,7-tetrahydro-1H-indol-1-yl) picolinic acid | H |
| 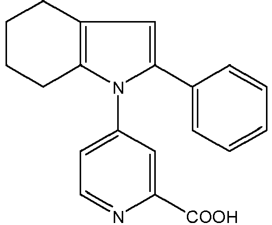 | 4-(2-phenyl-4,5 ,6,7-tetrahydro-1H-indol-1-yl) picolinic acid | I |
| 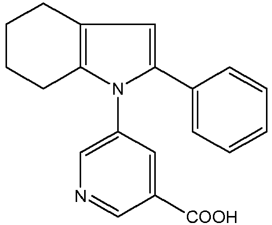 | 5-(2-phenyl-4,5 ,6,7-tetrahydro-1H-indol-1-yl) nicotinic acid | J |
| 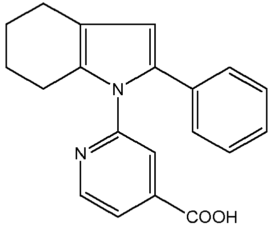 | 2-(2-phenyl-4,5 ,6,7-tetrahydro-1H-indol-1-yl) isonicotinic acid | K |
| 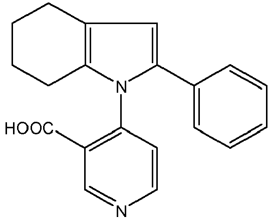 | 4-(2-phenyl-4,5 ,6,7-tetrahydro-1H-indol-1-yl) nicotinic acid | L |
| 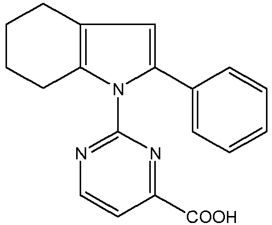 | 2-(2-phenyl-4,5,6,7-tetrahydro-1H-indol-1-yl) pyrimidine-4-carboxylic acid | M |
| 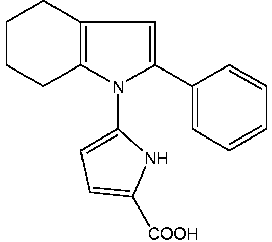 | 5-(2-phenyl-4,5 ,6,7-tetrahydro-1H-indol-1-yl)-1H-pyrrole-2-carboxylic acid | N |
| 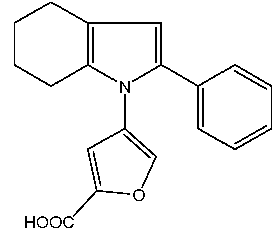 | 4-(2-phenyl-4,5,6,7-tetrahydro-1H-indol-1-yl) furan-2-carboxylic acid | O |
| 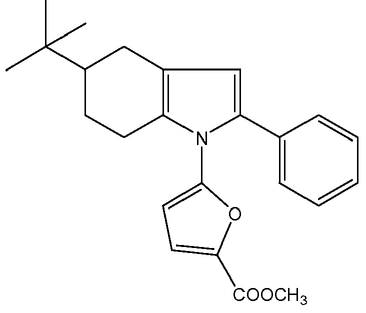 | 5-(5-tert-butyl-2-phenyl-4,5,6,7-tetrahydroindol-1-yl)furan-2-carboxylic acid methyl ester | P |
| 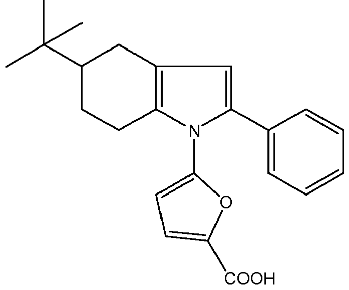 | 5-(5-tert-butyl-2-phenyl-4,5,6,7-tetrahydroindol-1-yl) furan-2-carboxylic acid | Q |
| 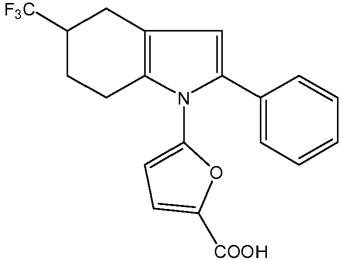 | 5-(2-phenyl-5-trifluoromethyl-4,5,6,7-tetrahydroindol-1-yl)-furan-2-carboxylic acid | R |
| 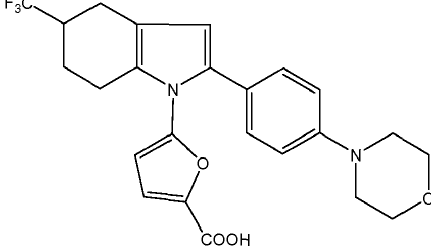 | 5-[2-(4-morpholin-4-yl-phenyl)-5-trifluoromethyl-4,5,6,7-tetrahydroindol-1-yl]-furan-2-carboxylic acid | S |
| 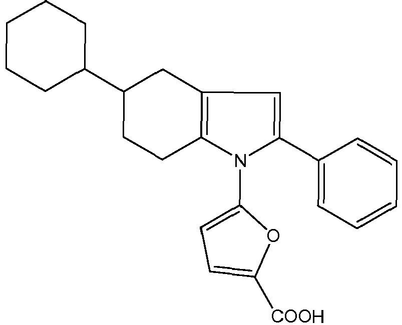 | 5-(5-cyclohexyl-2-phenyl-4,5,6,7-tetrahydroindol-1-yl)-furan-2-carboxylic acid | T |
| 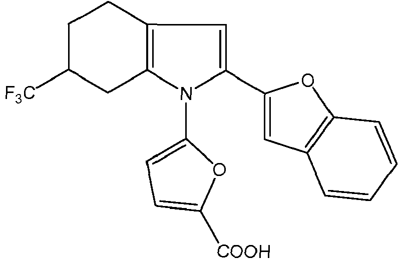 | 5-(2-benzofuran-2-yl-6-trifluoromethyl-4,5,6,7-tetrahydroindol-1-yl)-furan-2-carboxylic acid | U |
| 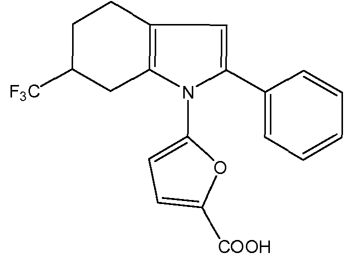 | 5-(2-phenyl-6-trifluoromethyl-4,5,6,7-tetrahydroindol-1-yl)-furan-2-carboxylic acid | V |
| 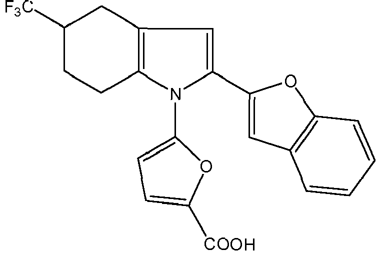 | 5-(2-benzofuran-2-yl-5-trifluoromethyl-4,5,6,7-tetrahydroindol-1-yl)-furan-2-carboxylic acid | W |
| 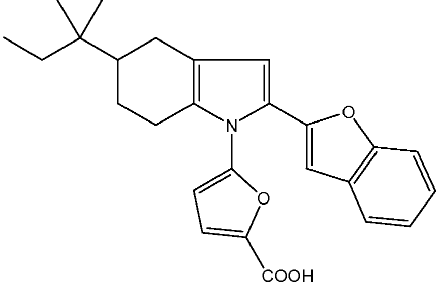 | 5-[2-benzofuran-2-yl-5-(1,1-dimethylpropyl)-4,5,6,7-tetrahydroindol-1-yl]-furan-2-carboxylic acid | X |
| 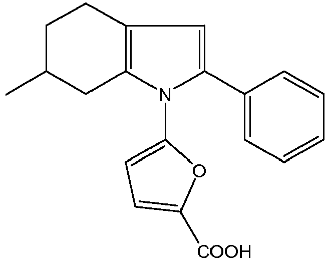 | 5-(6-methyl-2-phenyl-4,5,6,7-tetrahydroindol-1-yl) furan-2-carboxylic acid | Y |
| 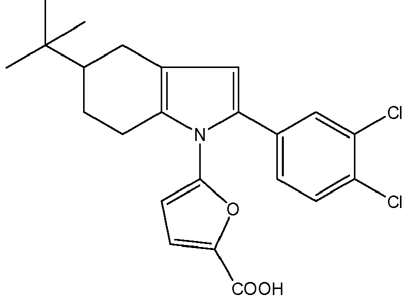 | 5-[5-tert-butyl-2-(3,4-dichlorophenyl)-4,5,6,7-tetrahydroindol-1-yl] furan-2-carboxylic acid | Z |
| 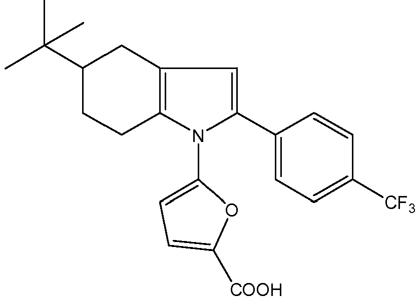 | 5-[5-tert-butyl-2-(4-trifluoromethylphenyl)-4,5,6,7-tetrahydroindol-1-yl]furan-2-carboxylic acid | AA |
| 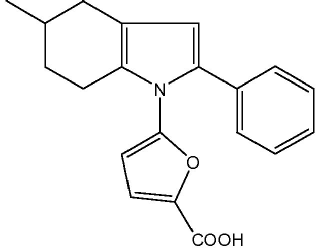 | 5-(5-methyl-2-phenyl-4,5,6,7-tetrahydroindol-1-yl) furan-2-carboxylic acid | AB |
| 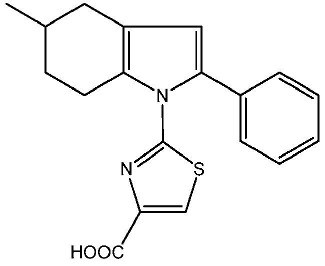 | 2-(5-methyl-2-phenyl-4,5,6,7-tetrahydroindol-1-yl) thiazole-4-carboxylic acid | AC |
| 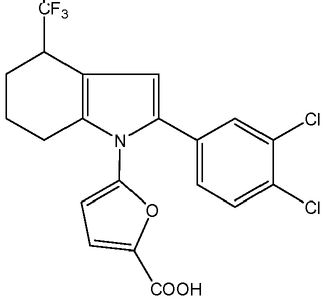 | 5-[2-(3,4-dichlorophenyl)-4-trifluoromethyl-4,5,6,7-tetrahydroindol-1-yl]-furan-2-carboxylic acid | AD |
| 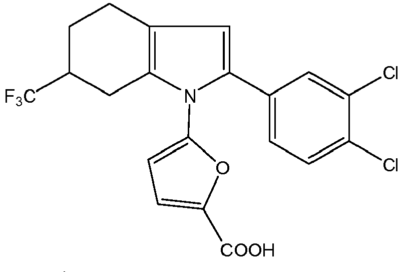 | 5-[2-(3,4-dichlorophenyl)-6-trifluoromethyl-4,5,6,7-tetrahydroindol-1-yl]-furan-2-carboxylic acid | AE |
| 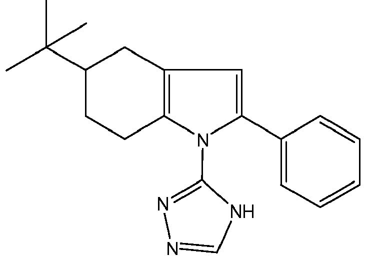 | 5-tert-butyl-2-phenyl-1-(4H-[1,2,4]triazol-3-yl)-4,5,6,7 -tetrahydro-1H-indole | AF |
| 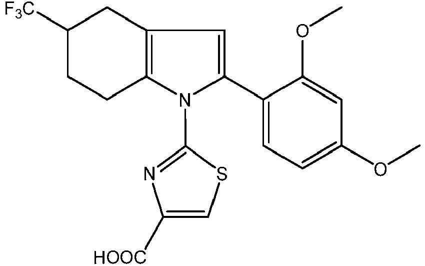 | 2-[2-(2,4-dimethoxyphenyl)-5-trifluoromethyl-4,5,6,7-tetrahydroindol-1-yl]-thiazole-4-carboxylic acid | AG |
| 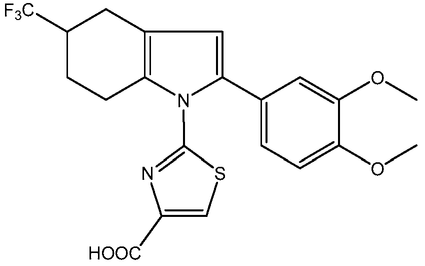 | 2-[2-(3,4-dimethoxyphenyl)-5-trifluoromethyl-4,5,6,7-tetrahydroindol-1-yl]-thiazole-4-carboxylic acid | AH |
| 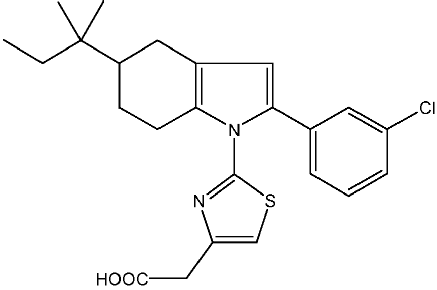 | {2-[2-(3-chlorophenyl)-5-(1,1-dimethylpropyl)-4,5,6,7-tetrahydroindol-1-yl]-thiazol-4-yl} acetic acid | AI |

In an embodiment, exemplary compounds include
3-[2-(3,4-dichloro-phenyl)-6-trifluoromethyl-4,5,6,7-tetrahydro-indol-1-yl]-benzoic acid;
3-(6-ethyl-2-phenyl-4,5,6,7-tetrahydroindol-1-yl)benzoic acid; and
3-(2-phenyl-5-trifluoromethyl-4,5,6,7-tetrahydroindol-1-yl)benzoic acid;
and pharmaceutically-acceptable salts of any of the foregoing.

In an embodiment, exemplary compounds include
5-[5-(1,1-dimethyl-propyl)-2-phenyl-4,5,6,7-tetrahydro-indol-1-yl]-furan-2-carboxylic acid;
5-[2-(3,4-dichloro-phenyl)-5-trifluoromethyl-4,5,6,7-tetrahydro-indol-1-yl]-furan-2-carboxylic acid;
{2-[5-(1,1-dimethylpropyl)-2-phenyl-4,5,6,7-tetrahydroindol-1-yl]-thiazol-4-yl} acetic acid;
6-(2-phenyl-4,5,6,7-tetrahydro-1H-indol-1-yl)picolinic acid;
4-(2-phenyl-4,5,6,7-tetrahydro-1H-indol-1-yl)picolinic acid;
5-(2-phenyl-4,5,6,7-tetrahydro-1H-indol-1-yl)nicotinic acid;
2-(2-phenyl-4,5,6,7-tetrahydro-1H-indol-1-yl)isonicotinic acid;
4-(2-phenyl-4,5,6,7-tetrahydro-1H-indol-1-yl)nicotinic acid;
2-(2-phenyl-4,5,6,7-tetrahydro-1H-indol-1-yl)pyrimidine-4-carboxylic acid;
5-(2-phenyl-4,5,6,7-tetrahydro-1H-indol-1-yl)-1H-pyrrole-2-carboxylic acid;
4-(2-phenyl-4,5,6,7-tetrahydro-1H-indol-1-yl)furan-2-carboxylic acid;
5-(5-tert-butyl-2-phenyl-4,5,6,7-tetrahydroindol-1-yl)furan-2-carboxylic acid methyl ester;
5-(5-tert-butyl-2-phenyl-4,5,6,7-tetrahydroindol-1-yl)furan-2-carboxylic acid;
5-(2-phenyl-5-trifluoromethyl-4,5,6,7-tetrahydroindol-1-yl)-furan-2-carboxylic acid;
5-[2-(4-morpholin-4-yl-phenyl)-5-trifluoromethyl-4,5,6,7-tetrahydroindol-1-yl]-furan-2-carboxylic acid;
5-(5-cyclohexyl-2-phenyl-4,5,6,7-tetrahydroindol-1-yl)-furan-2-carboxylic acid;
5-(2-benzofuran-2-yl-6-trifluoromethyl-4,5,6,7-tetrahydroindol-1-yl)-furan-2-carboxylic acid;
5-(2-phenyl-6-trifluoromethyl-4,5,6,7-tetrahydroindol-1-yl)-furan-2-carboxylic acid;
5-(2-benzofuran-2-yl-5-trifluoromethyl-4,5,6,7-tetrahydroindol-1-yl)-furan-2-carboxylic acid;
5-[2-benzofuran-2-yl-5-(1,1-dimethylpropyl)-4,5,6,7-tetrahydroindol-1-yl]-furan-2-carboxylic acid;
5-(6-methyl-2-phenyl-4,5,6,7-tetrahydroindol-1-yl) furan-2-carboxylic acid;
5-[5-tert-butyl-2-(3,4-dichlorophenyl)-4,5,6,7-tetrahydroindol-1-yl]furan-2-carboxylic acid;
5-[5-tert-butyl-2-(4-trifluoromethylphenyl)-4,5,6,7-tetrahydroindol-1-yl] furan-2-carboxylic acid;
5-(5-methyl-2-phenyl-4,5,6,7-tetrahydroindol-1-yl)furan-2-carboxylic acid;
2-(5-methyl-2-phenyl-4,5,6,7-tetrahydroindol-1-yl)thiazole-4-carboxylic acid;
5-[2-(3,4-dichlorophenyl)-4-trifluoromethyl-4,5,6,7-tetrahydroindol-1-yl]-furan-2-carboxylic acid;
5-[2-(3,4-dichlorophenyl)-6-trifluoromethyl-4,5,6,7-tetrahydroindol-1-yl]-furan-2-carboxylic acid;
5-tert-butyl-2-phenyl-1-(4H-[1,2,4]triazol-3-yl)-4,5,6,7-tetrahydro-1H-indole;
2-[2-(2,4-dimethoxyphenyl)-5-trifluoromethyl-4,5,6,7-tetrahydroindol-1-yl]-thiazole-4-carboxylic acid;
2-[2-(3,4-dimethoxyphenyl)-5-trifluoromethyl-4,5,6,7-tetrahydroindol-1-yl]-thiazole-4-carboxylic acid;
{2-[2-(3-chlorophenyl)-5-(1,1-dimethylpropyl)-4,5,6,7-tetrahydroindol-1-yl]-thiazol-4-yl} acetic acid;
and pharmaceutically-acceptable salts of any of the foregoing.

In an embodiment, the exemplary compounds may be used in a method of treating a neurodegenerative disease or disorder associated with compromised axonal function, including axonal dysfunction resulting from a defect in motor axonal growth or transport, in a human patient comprising, identifying a patient in need of such treatment and administering to said patient a therapeutically effective amount of said exemplary compound. In certain embodiments, the neurodegenerative disease or disorder associated with compromised axonal function is SMA, CMT2, HSP, hereditary sensory motor neuropathy and MS. In an embodiment, the disease is SMA.

Protected derivatives of the disclosed compounds also are contemplated. A variety of suitable protecting groups for use with the inventive compounds are disclosed. Other conventional protecting groups can be selected by those of skill in the art in consultation with Greene and Wuts, Protective Groups in Organic Synthesis; 3rd Ed.; John Wiley & Sons, New York, 1999.

The compounds of the present invention may be prepared in a variety of ways known to one skilled in the art of organic synthesis. The compounds of the present invention can be synthesized using the methods as described herein, together with synthetic methods known in the art of synthetic organic chemistry or variations thereon as appreciated by those skilled in the art.

Any active agents and reagents used in the following examples are either commercially available or can be prepared according to standard literature procedures by those skilled in the art of organic synthesis. In light of this disclosure, those of skill in the art will recognize that variations of these examples and other examples of the disclosed method would be possible without undue experimentation.

Some of the compounds of Formula I, for use in the invention may exist as single stereoisomers (*i.e*., essentially free of other stereoisomers), racemates, and/or mixtures of enantiomers and/or diastereomers. All such single stereoisomers, racemates and mixtures thereof are intended to be within the scope of the present invention. Preferably, the compounds that are optically active are used in optically pure form. Furthermore, some of the compound for use in the invention can exist as cis and trans geometric isomers. All such isomers and mixtures thereof are intended to be within the scope of the present invention.

Additionally, the formulas are intended to cover solvated as well as unsolvated forms of the identified structures. For example, Formula I includes compounds of the indicated structure in both hydrated and non-hydrated forms. Other examples of solvates include the structures in combination with isopropanol, ethanol, methanol, DMSO, ethyl acetate, acetic acid, or ethanolamine.

In addition to compounds of Formula I, the invention includes pharmaceutically acceptable prodrugs, pharmaceutically active metabolites, and pharmaceutically acceptable salts of such compounds.

Prodrugs and active metabolites of compound may be identified using routine techniques known in the art. See, e.g., Bertolini, G et al., J. Med. Chem., 40, 2011-2016 (1997); Shan, D. et al., J. Pharm. Sci., 86 (7), 756-767; Bagshawe K., Drug Dev. Res., 34, 220-230 (1995); Bodor N.; Advance in Drug Res., 13, 224-331 (1984); Bundgaard, H., Design of Prodrugs (Elsevier Press 1985); and Larsen, I. K., Design and Application of Prodrugs, Drug Design and Development (Krogsgaard-Larsen et al., eds., Harwood Academic Publishers, 1991).

### Definitions

As used herein, the term "alkyl" refers to a saturated aliphatic hydrocarbon including straight chain and branched chain groups. In an embodiment, the alkyl group has 1 to 20 carbon atoms (whenever it appears herein, a numerical range such as "1 to 20" refers to each integer in the given range; e.g., "1 to 20 carbon atoms" means that the alkyl group may consist of 1 carbon atom, 2 carbon atoms, 3 carbon atoms, etc. up to and including 20 carbon atoms). In certain embodiments, it is a medium size alkyl having 1 to 10 carbon atoms. In some embodiments, it is a lower alkyl having 1 to 6 carbon atoms, or 1 to 4 carbon atoms. The alkyl group may be substituted or unsubstituted. When substituted, the substituent group(s) may be one or more individually selected from cycloalkyl, aryl, heteroaryl, heteroalicyclic, hydroxy, alkoxy, aryloxy, mercapto, alkylthio, arylthio, cyano, halo, carbonyl, thiocarbonyl, O-carbamyl, N-carbamyl, O-thiocarbamyl, N-thiocarbamyl, C-amido, N-amido, C-carboxy, O-carboxy, cyanato, isocyanato, thiocyanato, isothiocyanato, nitro, silyl, and amino.

As used herein, the term "halo" refers to chloro, fluoro, bromo, and iodo.

As used herein, the term "hydro" refers to a hydrogen atom (-H group).

As used herein, the term "hydroxy" refers to an -OH group.

As used herein, the term "alkoxy" refers to both an -O-alkyl and an -O-cycloalkyl group, as defined herein. Lower alkoxy refers to -O-lower alkyl groups.

As used herein, the term "aryloxy" refers to both an -O-aryl and an -O-heteroaryl group, as defined herein.

As used herein, the term "mercapto" group refers to an -SH group.

As used herein, the term "alkylthio" group refers to both an S-alkyl and an -S-cycloalkyl group, as defined herein.

As used herein, the term "arylthio" group refers to both an -S-aryl and an -S-heteroaryl group, as defined herein.

As used herein, the term "carbonyl" group refers to a -C(=O)R" group, where R" is selected from the group consisting of hydro, alkyl, cycloalkyl, aryl, heteroaryl (bonded through a ring carbon) and heterocyclic (bonded through a ring carbon), as defined herein.

As used herein, the term "aldehyde" group refers to a carbonyl group where R" is hydro.

As used herein, the term "cycloketone" refer to a cycloalkyl group in which one of the carbon atoms which form the ring has a "=O" bonded to it; i.e. one of the ring carbon atoms is a -C(=O)-group.

As used herein, the term "thiocarbonyl" group refers to a -C(=S)R" group, with R" as defined herein.

As used herein, the term "O-carboxy" group refers to a R"C(=O)O-group, with R" as defined herein.

As used herein, the term "C-carboxy" group refers to a -C(=O)OR" groups with R" as defined herein.

As used herein, the term "ester" is a C-carboxy group, as defined herein, wherein R" is any of the listed groups other than hydro.

As used herein, the term "C-carboxy salt" refers to a -C(=O)O⁻ M⁺ group wherein M⁺ is selected from the group consisting of lithium, sodium, magnesium, calcium, potassium, barium, iron, zinc and quaternary ammonium.

As used herein, the term "acetyl" group refers to a -C(=O)CH₃ group.

As used herein, the term "carboxyalkyl" refers to -(CH₂)ᵣC(=O)OR" wherein r is 1-6 and R" is as defined above.

As used herein, the term "carboxyalkyl salt" refers to a -(CH₂)ᵣC(=O)O⁻M⁺ wherein M⁺ is selected from the group consisting of lithium, sodium, potassium, calcium, magnesium, barium, iron, zinc and quaternary ammonium.

As used herein, the term "carboxylic acid" refers to a C-carboxy group in which R" is hydro.

As used herein, the term "haloalkyl" refers to an alkyl group substituted with 1 to 6 halo groups, and may be a haloalkyl with a -CX₃ group wherein X is a halo group. The halo groups can be independently selected.

As used herein, the term "trihalomethanesulfonyl" refers to a X₃ CS(=O)₂- group with X as defined above.

As used herein, the term "cyano" refers to a -C≡N group.

As used herein, the term "cyanato" refers to a -CNO group.

As used herein, the term "isocyanato" refers to a -NCO group.

As used herein, the term "thiocyanato" refers to a -CNS group.

As used herein, the term "isothiocyanato" refers to a -NCS group.

As used herein, the term "sulfinyl" refers to a -S(=O)R" group, with R" as defined herein.

As used herein, the term "sulfonyl" refers to a -S(=O)₂ R" group, with R" as defined herein.

As used herein, the term "sulfonamido" refers to a -S(=O)₂ NR¹⁷R¹⁸, with R¹⁷ and R¹⁸ as defined herein.

As used herein, the term "trihalomethanesulfonamido" refers to a X₃CS(=O)₂ NR¹⁷ -group with X and R¹⁷ as defined herein.

As used herein, the term "O-carbamyl" refers to a -OC(=O)NR¹⁷ R¹⁸ group with R¹⁷ and R¹⁸ as defined herein.

As used herein, the term "N-carbamyl" refers to a R¹⁸ OC(=O)NR¹⁷- group, with R¹⁷ and R¹⁸ as defined herein.

As used herein, the term "O-thiocarbamyl" refers to a -OC(=S)NR¹⁷ R¹⁸ group with R¹⁷ and R¹⁸ as defined herein.

As used herein, the term "N-thiocarbamyl" refers to a R¹⁷OC(=S)NR¹⁸- group, with R¹⁷ and R¹⁸ as defined herein.

As used herein, the term "amino" refers to an -NR¹⁷ R¹⁸ group, with R¹⁷ and R¹⁸ both being hydro.

As used herein, the term "C-amido" refers to a -C(=O)NR¹⁷ R¹⁸ group with R¹⁷ and R¹⁸ as defined herein. An "N-amido" refers to a R¹⁷ C(=O)NR¹⁸- group with R¹⁷ and R¹⁸ as defined herein.

As used herein, the term "nitro" refers to a -NO₂ group.

As used herein, the term "quaternary ammonium" refers to a -⁺NR¹⁷ R¹⁸ R¹⁹ group wherein R¹⁷, R¹⁸, and R¹⁹ are independently selected from the group consisting of hydro and unsubstituted lower alkyl.

As used herein, the term "methylenedioxy, ethylenedioxy" refers to a -OCH₂O-group wherein the oxygen atoms are bonded to adjacent ring carbon atoms.

As used herein, the term "ethylenedioxy" refers to a -OCH₂CH₂O- group wherein the oxygen atoms are bonded to adjacent ring carbon atoms.

As used herein, the term "cycloalkyl" refers to an all-carbon monocyclic or fused ring (i.e., rings which share an adjacent pair of carbon atoms) group wherein one or more of the rings does not have a completely conjugated pi-electron system. Examples, without limitation, of cycloalkyl groups are cyclopropane, cyclobutane, cyclopentane, cyclopentene, cyclohexane, adamantane, cyclohexadiene, cycloheptane and, cycloheptatriene. A cycloalkyl group may be substituted or unsubstituted. When substituted, the substituent group(s) is preferably one or more individually selected from alkyl, aryl, heteroaryl, heterocyclic, hydroxy, alkoxy, aryloxy, mercapto, alkylthio, arylthio, cyano, halo, carbonyl, thiocarbonyl, carboxy, O-carbamyl, N-carbamyl, C-amido, N-amido, nitro, and amino.

As used herein, the term "heterocycle" or heterocyclic" refers to a saturated or partially saturated 3, 4, 5, 6 or 7-membered monocyclic, or 7, 8, 9, or 10-membered bicyclic ring system, which consists of carbon atoms and from one to four heteroatoms independently selected from the group consisting of O, N, and S, wherein the nitrogen and sulfur heteroatoms can be optionally oxidized, the nitrogen can be optionally quaternized, and including any bicyclic group in which any of the above-defined heterocyclic rings is fused to a benzene ring, and wherein the heterocyclic ring can be substituted on carbon or on a nitrogen atom if the resulting compound is stable. Non-limiting saturated or partially saturated heterocyclic groups include tetrahydrofuranyl, pyranyl, piperidinyl, piperazinyl, pyrrolidinyl, imidazolidinyl, imidazolinyl, indolinyl, isoindolinyl, quinuclidinyl, morpholinyl, isochromanyl, chromanyl, pyrazolidinyl, pyrazolinyl, tetronoyl and tetramoyl groups.. Example of "heterocycles" or "heterocyclic" rings also include, but are not limited to, morpholino, piperidyl, piperazinyl, pyrrolidinyl, thiomorpholino, homopiperazinyl, imidazolyl, imidazolidinyl, pyrazolidinyl, dioxanyl and dioxolanyl. "Heterocycle" can include heteroaryls when the pi-electron system of a heterocycle is completely conjugated.

As used herein, the term "aryl" refers to an all-carbon monocyclic or fused-ring polycyclic (i.e., rings which share adjacent pairs of carbon atoms) groups having a completely conjugated pi-electron system. Examples, without limitation, of aryl groups are phenyl, naphthalenyl and anthracenyl. The aryl group may be substituted or unsubstituted. When substituted, the substituted group(s) is preferably one or more selected from halo, trihalomethyl, alkyl, hydroxy, alkoxy, aryloxy, mercapto, alkylthio, arylthio, cyano, nitro, carbonyl, thiocarbonyl, C-carboxy, O-carboxy, O-carbamyl, N-carbamyl, O-thiocarbamyl, N-thiocarbamyl, C-amido, N-amido, sulfinyl, sulfonyl, S-sulfonamido, N-sulfonamido, trihalomethanesulfonamido, and amino.

As used herein, the term "heteroaryl" refers to groups having 5, 6, 7, 8, 9, 10, 11, 12, 13, or 14 ring atoms; 6, 10 or 14 pi electrons shared in a cyclic array; and containing carbon atoms and 1, 2 or 3 oxygen, nitrogen or sulfur heteroatoms. Non-limiting heteroaryl groups include thienyl (thiophenyl), benzo[b]thienyl, naphtho[2,3-b]thienyl, thianthrenyl, furyl (furanyl), isobenzofuranyl, chromenyl, xanthenyl, phenoxanthinyl, pyrrolyl, including without limitation 2H-pyrrolyl, imidazolyl, pyrazolyl, pyridyl (pyridinyl), including without limitation 2-pyridyl, 3-pyridyl, and 4-pyridyl, pyrazinyl, pyrimidinyl, pyridazinyl, indolizinyl, isoindolyl, 3H-indolyl, indolyl, indazolyl, purinyl, 4H-quinolizinyl, isoquinolyl, quinolyl, phthalzinyl, naphthyridinyl, quinozalinyl, cinnolinyl, pteridinyl, carbazolyl, beta-carbolinyl, phenanthridinyl, acrindinyl, perimidinyl, phenanthrolinyl, phenazinyl, isothiazolyl, phenothiazinyl, isoxazolyl, furazanyl, phenoxazinyl, 1,4-dihydroquinoxaline-2,3-dione, 7 aminoisocoumarin, pyrido[1,2-a]pyrimidin-4-one, pyrazolo[1,5-a]pyrimidinyl, including without limitation pyrazolo[1,5-a]pyrimidin-3-yl, 1,2-benzoisoxazol-3-yl, benzimidazolyl, 2-oxindolyl and 2 oxobenzimidazolyl. Where the heteroaryl group contains a nitrogen atom in a ring, such nitrogen atom may be in the form of an N-oxide, e.g., a pyridyl N oxide, pyrazinyl N-oxide and pyrimidinyl N-oxide. When substituted, the substituted group(s) is preferably one or more selected from alkyl, cycloalkyl, halo, trihalomethyl, hydroxy, alkoxy, aryloxy, mercapto, alkylthio, arylthio, cyano, nitro, carbonyl, thiocarbonyl, sulfonamido, carboxy, sulfinyl, sulfonyl, O-carbamyl, N-carbamyl, O-thiocarbamyl, N-thiocarbamyl, C-amido, N-amido, and amino.

As used herein, the term "preventing an increase in a symptom" refers to both not allowing a symptom to increase or worsen, as well as reducing the rate of increase in the symptom. For example, a symptom can be measured as the amount of particular disease marker, *i.e.,* a protein. In another example the symptom can be cognitive decline. Preventing an increase, according to the definition provided herein, means that the amount of symptom (*e.g.,* protein or cognitive decline) does not increase or that the rate at which it increases is reduced.

As used herein, the term "treating a neurodegenerative disorder" refers to a slowing of or a reversal of the progress of the disorder. Treating a neurodegenerative disorder includes treating a symptom and/or reducing the symptoms of the disorder.

As used herein, the term "preventing a neurodegenerative disorder" refers to a slowing of the disorder or of the onset of the disorder or the symptoms thereof. Preventing a neurodegenerative disorder can include stopping the onset of the disorder or symptoms thereof.

As used herein, the term "unit dosage form" refers to a physically discrete unit, such as a capsule or tablet suitable as a unitary dosage for a human patient. Each unit contains a predetermined quantity of a compound of Formula I, which was discovered or believed to produce the desired pharmacokinetic profile which yields the desired therapeutic effect. The dosage unit is composed of a compound of Formula I in association with at least one pharmaceutically acceptable carrier, salt, excipient, or combination thereof.

As used herein, the term "dose" or "dosage" refers the amount of active ingredient that an individual takes or is administered at one time. For example, an 800 mg dose of a compound of Formula I refers to, in the case of a twice-daily dosage regimen, a situation where the individual takes 800 mg of a compound of Formula I twice a day, e.g., 800 mg in the morning and 800 mg in the evening. The 800 mg of a compound of Formula I dose can be divided into two or more dosage units, *e.g.,* two 400 mg dosage units of a compound of Formula I in tablet form or two 400 mg dosage units of a compound of Formula I in capsule form.

"A pharmaceutically acceptable prodrug" is a compound that may be converted under physiological conditions or by solvolysis to the specified compound or to a pharmaceutically acceptable salt of such compound.

"A pharmaceutically active metabolite" is intended to mean a pharmacologically active product produced through metabolism in the body of a specified compound or salt thereof. Metabolites of a compound may be identified using routine techniques known in the art and their activities determined using tests such as those described herein.

"A pharmaceutically acceptable salt" is intended to mean a salt that retains the biological effectiveness of the free acids and bases of the specified compound and that is not biologically or otherwise undesirable. A compound for use in the invention may possess a sufficiently acidic, a sufficiently basic, or both functional groups, and accordingly react with any of a number of inorganic or organic bases, and inorganic and organic acids, to form a pharmaceutically acceptable salt. Exemplary pharmaceutically acceptable salts include those salts prepared by reaction of the compounds of the present invention with a mineral or organic acid or an inorganic base, such as salts including sulfates, pyrosulfates, bisulfates, sulfites, bisulfites, phosphates, monohydrophosphates, dihydrophosphates, metaphosphates, pyrophosphates, chlorides, bromides, iodides, acetates, propionates, decanoates, caprylates, acrylates, formates, isobutyrates, caproates, heptanoates, propiolates, oxalates, malonates, succinates, suberates, sebacates, fumarates, maleates, butyne-1,4 dioates, hexyne-1,6-dioates, benzoates, chlorobenzoates, methylbenzoates, dinitrobenzoates, hydroxybenzoates, methoxybenzoates, phthalates, sulfonates, xylenesulfonates, phenylacetates, phenylpropionates, phenylbutyrates, citrates, lactates, gamma-hydroxybutyrates, glycollates, tartrates, methane-sulfonates, propanesulfonates, naphthalene-1-sulfonates, naphthalene-2-sulfonates, and mandelates.

### Dosages, formulations, and route of administration

The active compounds of this invention are typically administered in combination with a pharmaceutically acceptable carrier through any appropriate routes such as parenteral, oral, or topical administration, in a therapeutically (or prophylactically) effective amount according to the methods set forth above. A preferred route of administration for use in the invention is oral administration.

Generally, the toxicity profile and therapeutic efficacy of the therapeutic agents can be determined by standard pharmaceutical procedures in suitable cell models or animal models. As is known in the art, the LD50 represents the dose lethal to about 50% of a tested population. The ED50 is a parameter indicating the dose therapeutically effective in about 50% of a tested population. Both LD50 and ED50 can be determined in cell models and animal models. In addition, the IC50 may also be obtained in cell models and animal models, which stands for the circulating plasma concentration that is effective in achieving about 50% of the maximal inhibition of the symptoms of a disease or disorder. Such data may be used in designing a dosage range for clinical trials in humans. Typically, as will be apparent to skilled artisans, the dosage range for human use should be designed such that the range centers around the ED50 and/or IC50, but remains significantly below the LD50 dosage level, as determined from cell or animal models.

Typically, the compounds and compositions for use in the invention can be effective at an amount of from about 0.05 mg to about 4000 mg per day, preferably from about 0.1 mg to about 2000 mg per day. However, the amount can vary with the body weight of the patient treated and the state of disease conditions. The active ingredient may be administered at once, or may be divided into a number of smaller doses to be administered at predetermined intervals of time.

In the case of combination therapy, a therapeutically effective amount of another therapeutic compound can be administered in a separate pharmaceutical composition, or alternatively included in the pharmaceutical composition according to the present invention. The pharmacology and toxicology of other therapeutic compositions are known in the art. See e.g., Physicians Desk Reference, Medical Economics, Montvale, NJ; and The Merck Index, Merck & Co., Rahway, NJ. The therapeutically effective amounts and suitable unit dosage ranges of such compounds used in the art can be equally applicable in the present invention.

It should be understood that the dosage ranges set forth above are exemplary only and are not intended to limit the scope of this invention. The therapeutically effective amount for each active compound can vary with factors including but not limited to the activity of the compound used, stability of the active compound in the patient's body, the severity of the conditions to be alleviated, the total weight of the patient treated, the route of administration, the ease of absorption, distribution, and excretion of the active compound by the body, the age and sensitivity of the patient to be treated, and the like, as will be apparent to a skilled artisan. The amount of administration can also be adjusted as the various factors change over time.

The active compounds can also be administered parenterally in the form of solution or suspension, or in lyophilized form capable of conversion into a solution or suspension form before use. In such formulations, diluents or pharmaceutically acceptable carriers such as sterile water and physiological saline buffer can be used. Other conventional solvents, pH buffers, stabilizers, anti-bacterial agents, surfactants, and antioxidants can all be included. For example, useful components include sodium chloride, acetate, citrate or phosphate buffers, glycerin, dextrose, fixed oils, methyl parabens, polyethylene glycol, propylene glycol, sodium bisulfate, benzyl alcohol, ascorbic acid, and the like. The parenteral formulations can be stored in any conventional containers such as vials and ampules.

Routes of topical administration include nasal, bucal, mucosal, rectal, or vaginal applications. For topical administration, the active compounds can be formulated into lotions, creams, ointments, gels, powders, pastes, sprays, suspensions, drops and aerosols. Thus, one or more thickening agents, humectants, and stabilizing agents can be included in the formulations. Examples of such agents include, but are not limited to, polyethylene glycol, sorbitol, xanthan gum, petrolatum, beeswax, or mineral oil, lanolin, squalene, and the like. A special form of topical administration is delivery by a transdermal patch. Methods for preparing transdermal patches are disclosed, e.g., in Brown, et al., Annual Review of Medicine, 39:221-229 (1988).

Subcutaneous implantation for sustained release of the active compounds may also be a suitable route of administration. This entails surgical procedures for implanting an active compound in any suitable formulation into a subcutaneous space, e.g., beneath the anterior abdominal wall. See, *e.g.,* Wilson et al., J. Clin. Psych. 45:242-247 (1984). Hydrogels can be used as a carrier for the sustained release of the active compounds. Hydrogels are generally known in the art. They are typically made by crosslinking high molecular weight biocompatible polymers into a network that swells in water to form a gel like material. Preferably, hydrogels are biodegradable or biosorbable. For purposes of this invention, hydrogels made of polyethylene glycols, collagen, or poly(glycolic-co-L-lactic acid) may be useful. See, *e.g.,* Phillips et al., J. Pharmaceut. Sci. 73:1718-1720 (1984).

The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. When the dosage unit form is a capsule, it can contain, in addition to material of the above type, a liquid carrier such as a fatty oil. In addition, dosage unit forms can contain various other materials which modify the physical form of the dosage unit, for example, coatings of sugar, shellac, or other enteric agents.

Soft gelatin capsules can be prepared in which capsules contain a mixture of the active ingredient and vegetable oil or non-aqueous, water miscible materials such as, for example, polyethylene glycol and the like. Hard gelatin capsules may contain granules of the active ingredient in combination with a solid, pulverulent carrier, such as, for example, lactose, saccharose, sorbitol, mannitol, potato starch, corn starch, amylopectin, cellulose derivatives, or gelatin.

Tablets for oral use are typically prepared in the following manner, although other techniques may be employed. The solid substances are ground or sieved to a desired particle size, and the binding agent is homogenized and suspended in a suitable solvent. The active ingredient and auxiliary agents are mixed with the binding agent solution. The resulting mixture is moistened to form a uniform suspension. The moistening typically causes the particles to aggregate slightly, and the resulting mass is gently pressed through a stainless steel sieve having a desired size. The layers of the mixture are then dried in controlled drying units for determined length of time to achieve a desired particle size and consistency. The granules of the dried mixture are gently sieved to remove any powder. To this mixture, disintegrating, anti-friction, and anti-adhesive agents are added. Finally, the mixture is pressed into tablets using a machine with the appropriate punches and dies to obtain the desired tablet size. The operating parameters of the machine may be selected by the skilled artisan.

If the compound for use in the invention is a base, the desired pharmaceutically acceptable salt may be prepared by any suitable method available in the art, for example, treatment of the free base with an inorganic acid, such as hydrochloric acid, hydrobromic acid, sulfuric acid, nitric acid, phosphoric acid and the like, or with an organic acid, such as acetic acid, maleic acid, succinic acid, mandelic acid, fumaric acid, malonic acid, pyruvic acid, oxalic acid, glycolic acid, salicylic acid, a pyranosidyl acid, such as glucuronic acid or galacturonic acid, an alpha-hydroxy acid, such as citric acid or tartaric acid, an amino acid, such as aspartic acid or glutamic acid, an aromatic acid, such as benzoic acid or cinnamic acid, a sulfonic acid, such as p-toluenesulfonic acid or ethanesulfonic acid, or the like.

If the compound for use in the invention is an acid, the desired pharmaceutically acceptable salt may be prepared by any suitable method, for example, treatment of the free acid with an inorganic or organic base, such as an amine (primary, secondary or tertiary), an alkali metal hydroxide or alkaline earth metal hydroxide, or the like. Illustrative examples of suitable salts include organic salts derived from amino acids, such as glycine and arginine, ammonia, primary, secondary, and tertiary amines, and cyclic amines, such as piperidine, morpholine and piperazine, and inorganic salts derived from sodium, calcium, potassium, magnesium, manganese, iron, copper, zinc, aluminum and lithium. These substituents may optionally be further substituted with a substituent selected from such groups.

### Compound Preparation

Representative synthetic schemes and experimental descriptions for the compounds for use in the methods of the invention are given in the Examples below.

General: Chemicals were purchased from standard commercial vendors and used as received unless otherwise noted. "Degassed" means reduced pressure then nitrogen gas for three cycles. Abbreviations are consistent with those in the ACS Style Guide., plus: satd (saturated), DCM (dichloromethane), pRPLC (preparative HPLC), "dry" glassware means oven/desiccator dried. Solvents were ACS grade unless otherwise noted. Analytical TLC plates (Silica Gel 60 F254, EM Science, Gibbstown, NJ, or Merck # 5715) were used to follow the course of reactions, and the MPLC system used for purifications was from Isco (Foxy Jr fraction collector, UA-6 detector), using Isco silica gel flash columns (10 or 40 g). ¹H NMR spectra in CDCl₃, CD₃OD, and/or d6-DMSO were recorded on either a Varian Mercury 400 MHz or Brucker ARX-300 MHz instrument and chemical shifts are expressed in parts per million (ppm, δ) relative to TMS as the internal standard. Mass spectra were obtained on a Thermo Finnigan LCQ-Deca (injection volume 5 uL, XTerra MS-C₁₈ 3.5 µm 2.1 x 50mm column, XTerra MS-C₁₈ 5 µm 2.1 x 20mm guard column), ESI source unless otherwise specified, analytical HPLC was performed on an HP1050 (injection volume 5 µl, XTerra RP-C₁₈ 5 µm 4.6 x 250 mm column, with an XTerra MS-C₁₈ 5 µm 2.1 x 20mm guard column), and preparative HPLC was performed on an Agilent 1100 Prep-LC with various columns and conditions depending on the compound. GCMS was performed on either an Agilent Technology 6890N or Shimadzu QP5000/17A instrument. Yields are unoptimized.

### Example 1: Synthesis of a Representative Compound

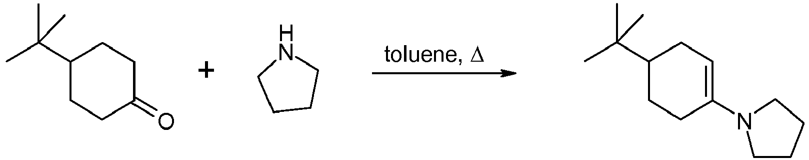

**1-(4-tert-butylcyclohex-1-enyl) pyrrolidine:** A 50 mL round-bottomed flask containing 4-tert-butylcyclohexanone (6.01 gm) in anhydrous toluene (20 mL) was fitted with a Dean-Stark trap containing 3A molecular sieves, reflux condenser and a heating mantle. Pyrrolidine (6.00 mL) was added, and the solution heated to reflux for 18 hr. The solvent was evaporated and the crude product was used directly for the next reaction.

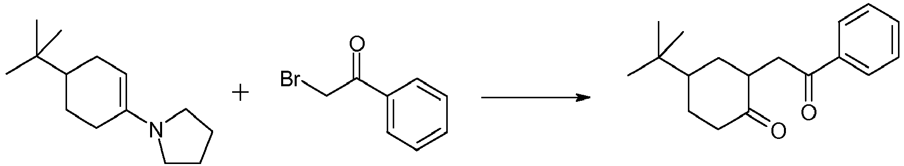

**4-tert-butyl-2-(2-oxo-2-phenylethyl)-cyclohexanone:** To a 250-mL round-bottomed flask containing 3.3 mL of 1-(4-tert-butylcyclohex-1-enyl) pyrrolidine was added 100 mL anhydrous DMF, under nitrogen. The flask was fitted with an addition funnel containing 2-bromoacetophenone (4.12 gm) in 35 mL anhydrous DMF, which was dripped into the enamine solution over 60 min. This solution was stirred at ambient temperature for 10 hr, then 90 mL water was added to the solution and it was stirred for another 11 hr, under nitrogen. The solution was then extracted twice with ethyl acetate and water, the organic layers combined and further washed with water (3x), dried over sodium sulfate, filtered and rotovapped down to give a yellow oil. The oil was purified by MPLC using 10% ethyl acetate/hexanes.

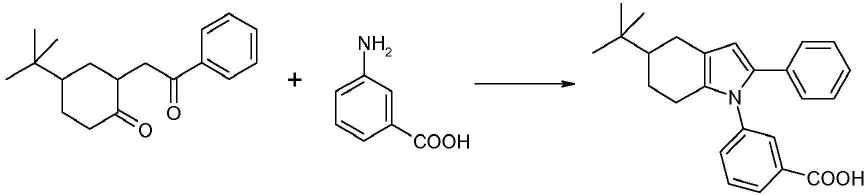

**3-(5-tert-butyl-2-phenyl-4,5,6,7-tetrahydroindol-1-yl)benzoic acid:** A solution of 4-tert-butyl-2-(2-oxo-2-phenylethyl)-cyclohexanone (0.219 gm) in glacial acetic acid (3.0 mL) in a 25-mL round-bottomed flask, under nitrogen, was fitted with a heating mantle and reflux condenser. To this solution was added 3-aminobenzoic acid (0.138 gm), which was then heated at 110C for 3 hr. The solution was cooled to ambient temperature, 8 mL water was added, and the suspension was stirred 18 hr under nitrogen. The solid was filtered, washed with water, and recrystallized in acetonitrile to provide 0.123 gm of the pure product.

Example 2. Following generally the experimental details provided for the Representative Compound above, and the following synthetic routes described below, the compounds of Formula I and their analogs in Table 1 were synthesized.

Allen, et al, J. Med. Chem. 1976, 19(2), 318-325. 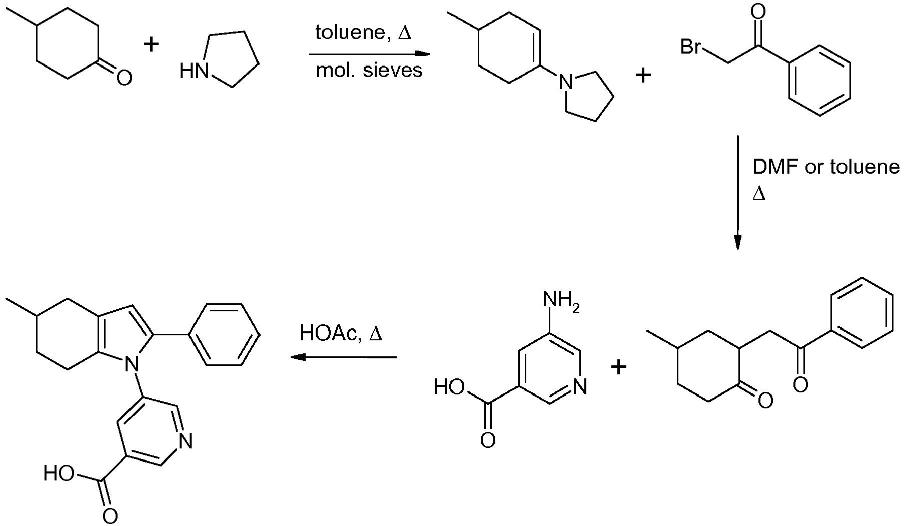
Route B: Murakami, et al, Chem. Pharm. Bull. 1995, 43(8), 1281-1286. 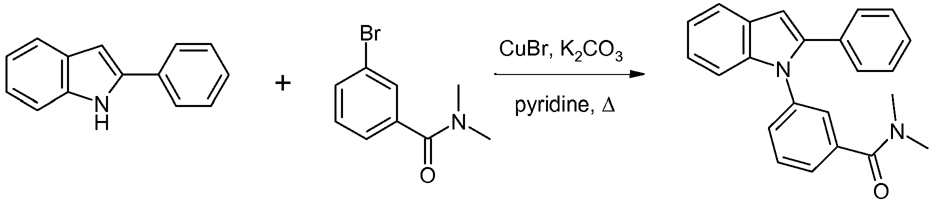
Route C: Allen, et al, J. Med. Chem. 1976, 19(2), 318-325.

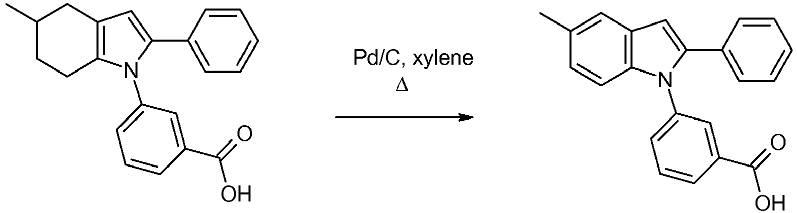

**Table 2: Starting materials for Exemplary compounds of Formula I and analogs thereof:**

| **compound abbreviation** | **SM ketone** | **alpha-bromo ketone** | **amine/aniline** |
|---|---|---|---|
| A | 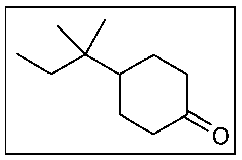 | 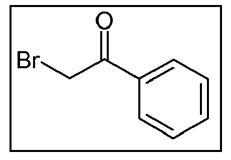 | 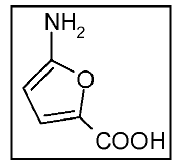 |
| B | 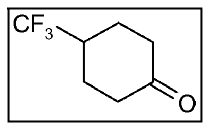 | 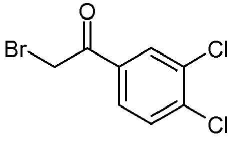 | 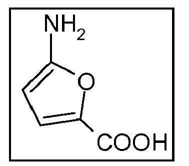 |
| C | 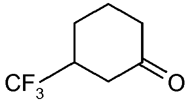 | 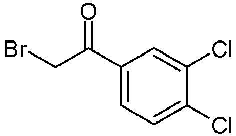 | 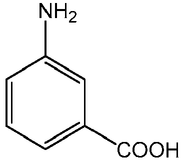 |
| D | 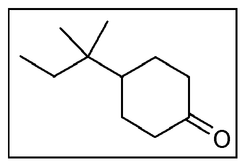 | 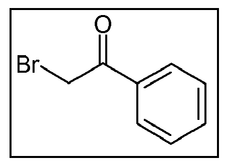 | 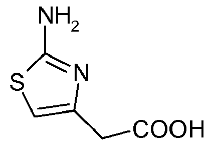 |
| E | 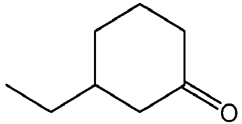 | 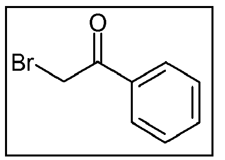 | 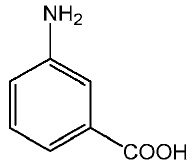 |
| F | 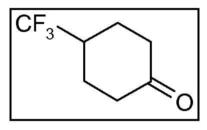 | 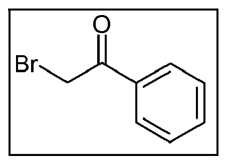 | 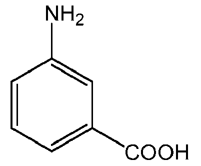 |
| G | 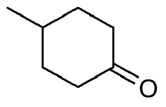 | 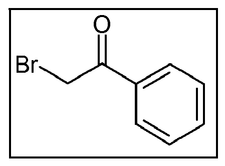 | 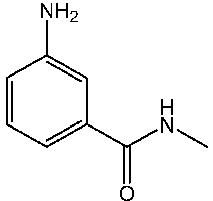 |
| H | 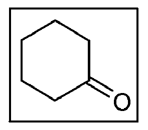 | 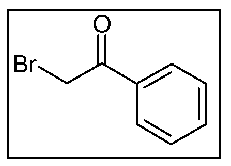 | 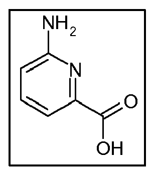 |
| I | 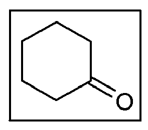 | 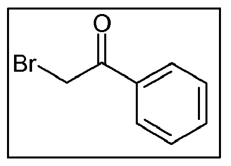 | 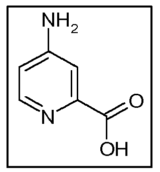 |
| J | 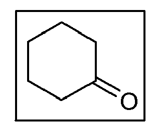 | 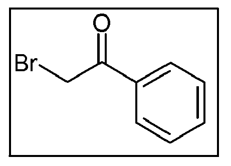 | 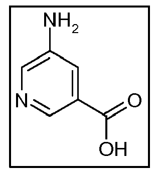 |
| K | 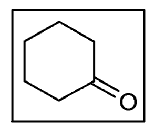 | 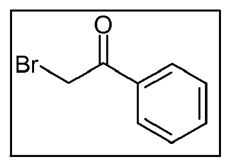 | 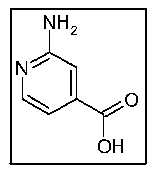 |
| L | 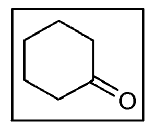 | 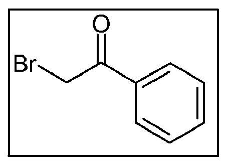 | 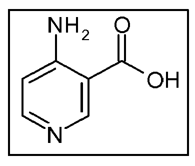 |
| M | 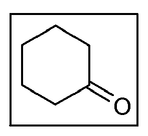 | 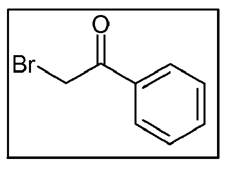 | 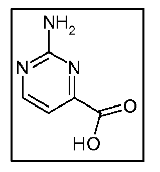 |
| N | 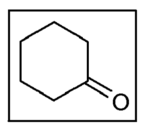 | 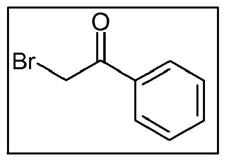 | 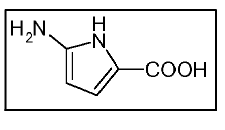 |
| O | 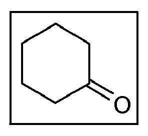 | 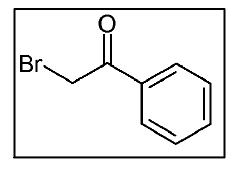 | 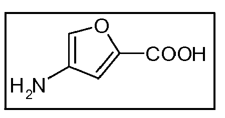 |
| P | 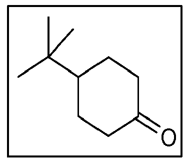 | 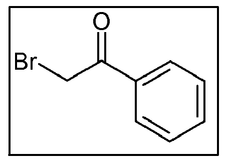 | 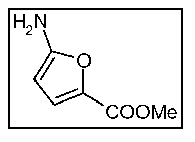 |
| Q | 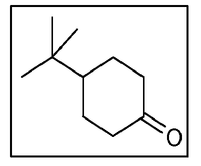 | 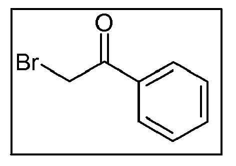 | 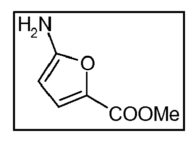 |
| R | 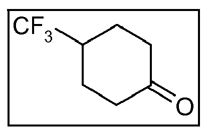 | 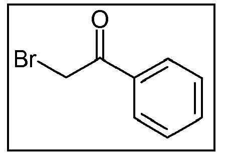 | 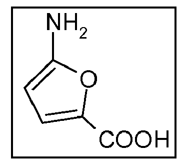 |
| S | 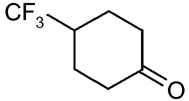 | 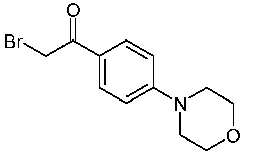 | 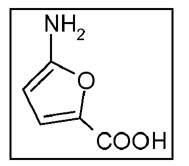 |
| T | 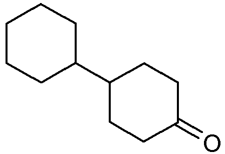 | 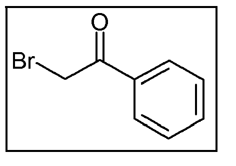 | 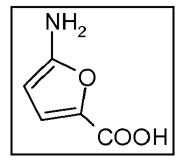 |
| U | 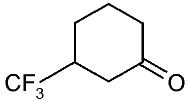 | 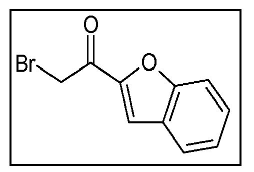 | 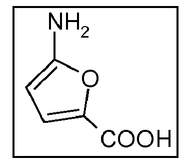 |
| V | 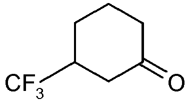 | 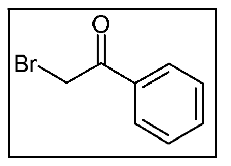 | 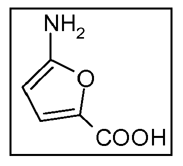 |
| W | 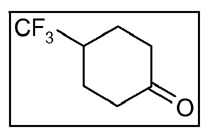 | 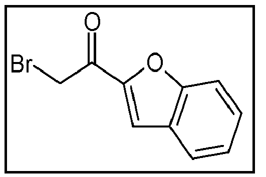 | 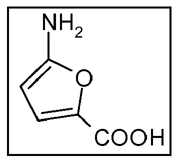 |
| X | 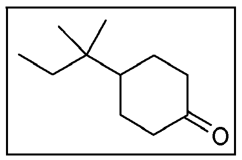 | 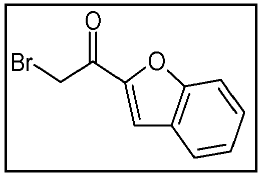 | 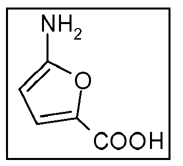 |
| Y | 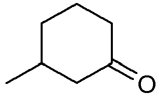 | 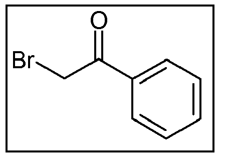 | 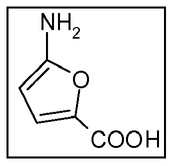 |
| Z | 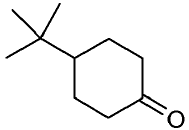 | 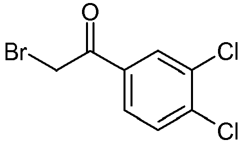 | 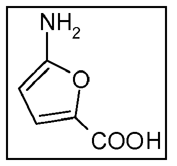 |
| AA | 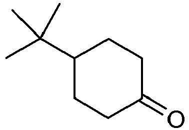 | 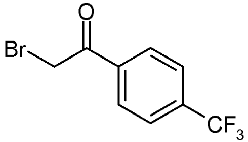 | 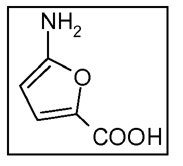 |
| AB | 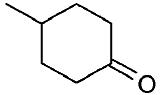 | 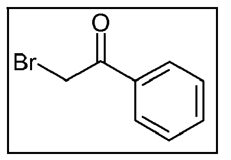 | 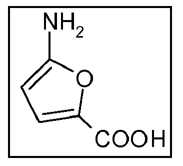 |
| AC | 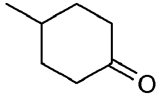 | 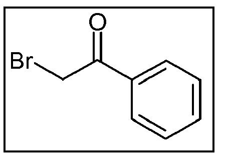 | 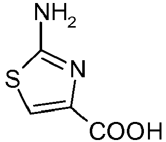 |
| AD | 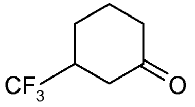 | 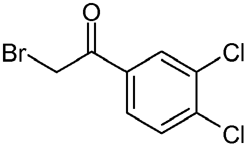 | 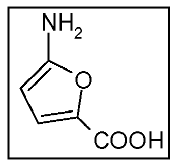 |
| AE | 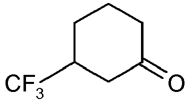 | 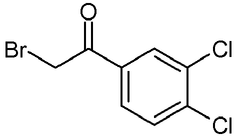 | 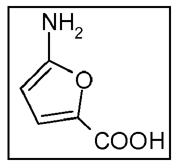 |
| AF | 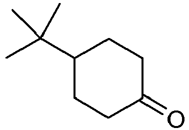 | 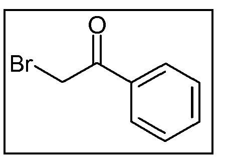 | 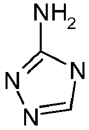 |
| AG | 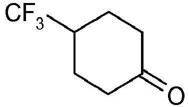 | 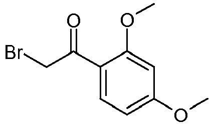 | 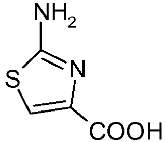 |
| AH | 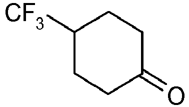 | 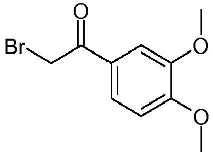 | 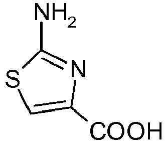 |
| AI | 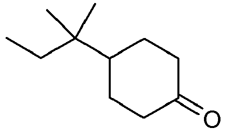 | 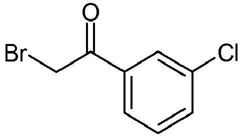 | 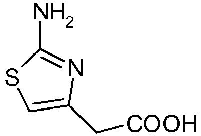 |

The Exemplary Compounds shown in Table 1 were synthesized via synthetic route A, following generally the experimental details provided for the Representative Compound above.
Compound A. Name: 5-[5-(1,1-dimethylpropyl)-2-phenyl-4,5,6,7-tetrahydroindol-1-yl]-furan-2-carboxylic acid. 1H NMR, δ; CDCl3; 7.10 - 7.30 (m, 6H), 6.21 (s, 1H), 5.95 (d, 1H), 2.56 - 2.64 (m, 2H), 2.54 (d, 1H), 2.32 (t, 1H), 2.00 (br d, 1H), 1.59 (td, 1H), 1.30 - 1.48 (m, 3H), 0.89 (s, 3H), 0.86 (s, 3H), 0.84 (t, 3H). MS; pos. mode 378 (M + H); neg. mode 376 (M - H).
Compound B. Name: 5-[2-(3,4-dichlorophenyl)-5-trifluoromethyl-4,5,6,7-tetrahydroindol-1-yl]-furan-2-carboxylic acid. 1H NMR, CDCl3; 7.24 - 7.33 (m, 3H), 6.89 (dd, 1H), 6.26 (s, 1H), 6.10 (d, 1H), 2.83 (dd, 1H), 2.55 - 2.70 (m, 3H), 2.37 - 2.53 (m, 1H), 2.23 (br d, 1H), 1.70 - 1.83 (m, 1H). MS; pos. mode 444 (M + H).
Compound C. Name: 3-[2-(3,4-dichlorophenyl)-6-trifluoromethyl-4,5,6,7-tetrahydroindol-1-yl] benzoic acid. 1H NMR, CDCl3; 8.11 (dt, 1H), 7.88 (br s, 1H), 7.52 (t, 1H), 7.33 (br s, 1H), 7.15 (d, 1H), 7.17 (d, 1H), 6.59 (dd, 1H), 6.29 (s, 1H), 2.78 (dd, 1H), 2.40 - 2.70 (m, 4H), 2.15 - 2.25 (m, 1H), 1.73 (qd, 1H). MS; pos. mode 454 (M + H).
Compound D. Name: {2-[5-(1,1-dimethylpropyl)-2-phenyl-4,5,6,7-tetrahydroindol-1-yl]-thiazol-4-yl} acetic acid. 1H NMR, δ; CDCl3; 7.22 - 7.30 (m, 3H), 7.16 - 7.22 (m, 2H), 6.96 (X of ABX, 1H), 6.22 (s, 1H), 3.83 (A of ABX, 1H), 3.81 (B of ABX, 1H), 2.64 - 2.74 (m, 2H), 2.57 (dd, 1H), 2.28 - 2.38 (m, 1H), 1.98 - 2.06 (m, 1H), 1.60 (tdd, 1H), 1.30 - 1.46 (m, 3H), 0.89 (s, 3H), 0.89 (s, 3H), 0.84 (t, 3H). MS; pos. mode 409 (M + H); neg. mode 407 (M - H).
Compound E. Name: 3-(6-ethyl-2-phenyl-4,5,6,7-tetrahydroindol-1-yl)benzoic acid. 1H NMR, CDCl3; 8.02 (dt, 1H), 7.94 (br s, 1H), 7.42 (t, 1H), 7.30 (br d, 1H), 7.11 - 7.18 (m, 2H), 7.02 - 7.11 (m, 3H), 6.26 (s, 1H), 2.54 - 2.74 (m, 2H), 2.44 (dd, 1H), 2.10 - 2.20 (m, 1H), 1.94 (br d, 1H), 1.65 (br s, 1H), 1.30 - 1.50 (m, 3H), 0.91 (t, 3H). MS; pos. mode 346 (M + H); neg. mode 344 (M - H).
Compound F. Name: 3-(2-phenyl-5-trifluoromethyl-4,5,6,7-tetrahydroindol-1-yl) benzoic acid. 1H NMR, CDCl3; 8.03 (dt, 1H), 7.93 (br s, 1H), 7.43 (t, 1H), 7.26 - 7.30 (m, 1H), 7.08 - 7.20 (m, 3H), 7.04 - 7.06 (m, 2H), 6.28 (s, 1H), 2.91 (dd, 1H), 2.55 - 2.74 (m, 2H), 2.45 - 2.55 (m, 2H), 2.15 - 2.25 (m, 1H), 1.75 (qd, 1H). MS; pos. mode 386 (M + H).
Compound G. Name: N-methyl-3-(5-methyl-2-phenyl-4,5,6,7-tetrahydroindol-1-yl) benzamide. 1H NMR, CDCl3; 7.7 (dm, 1H); 7.5 (br. s, 1H); 7.4 (t, 1H); 7.2 (m, 1H); 7.0 - 7.1 (m, 5H); 6.2 (s, 1H); 5.9 (br. s, 1H); 3.0 (d, 3H); 2.7 (dd, 1H); 2.5 (m, 1H); 2.4 (m, 1H); 2.2 (m, 1H); 1.9 (m, 2H); 1.4 (m, 1H); 1.1 (d, 3H). MS; pos. mode 345 (M + H).
Compound P. Name: 5-(5-tertButyl-2-phenyl-4,5,6,7-tetrahydroindol-1-yl)furan-2-carboxylic acid methyl ester. 1H NMR, δ; CDCl3; 7.1 - 7.3 (m, 6H, ArH); 6.2 (s, 1H); 6.0 (d, 1H); 3.9 (s, 3H); 2.6 (m, 3H); 2.3 (m, 1H); 2.0 (m, 1H); 1.3 - 1.5 (m, 2H); 1.0 (s, 9H). MS; pos. mode 378 (M + H).
Compound Q. Name: 5-(5-tertButyl-2-phenyl-4,5,6,7-tetrahydroindol-1-yl)furan-2-carboxylic acid. 1H NMR, δ; CDCl3; 7.1 - 7.3 (m, 6H, ArH); 6.2 (s, 1H); 6.0 (d, 1H); 2.6 (m, 2H); 2.4 -2.5 (m, 2H); 2.0 (m, 1H); 1.5 (m, 2H); 1.0 (s, 9H). MS; pos. mode 364 (M + H); neg. mode 362 (M - H).
Compound R. Name: 5-(2-phenyl-5-trifluoromethyl-4,5,6,7-tetrahydroindol-1-yl)-furan-2-carboxylic acid. 1H NMR, δ; CDCl3; 7.20 - 7.32 (m, 4H), 7.14 (d, 2H), 6.23 (s, 1H), 5.99 (d, 1H), 2.84 (dd, 1H), 2.58 - 2.73 (m, 3H), 2.46 (br s, 1H), 2.20 - 2.38 (m, 1H), 1.70 - 1.85 (m, 1H). MS; pos. mode 376 (M + H).
Compound S. Name: 5-[2-(4-morpholin-4-yl-phenyl)-5-trifluoromethyl-4,5,6,7-tetrahydroindol-1-yl]-furan-2-carboxylic acid. 1H NMR, δ; DMSO-d6; 7.27 (s, 1H); 6.97 (d, 2H); 6.84 (d, 2H); 6.51 (s, 1H); 6.22 (s, 1H); 3.7 (t, 4H); 3.1 (t, 4H); 2.73 (m, 2H); 2.5 (m, 3H); 2.12 (m, 1H); 1.64 (m, 1H). MS; pos. mode 461 (M + H); neg. mode 459 (M - H).
Compound T. Name: 5-(5-cyclohexyl-2-phenyl-4,5,6,7-tetrahydroindol-1-yl)-furan-2-carboxylic acid. 1H NMR, δ; CDCl3; CDCl3; 7.15 - 7.28 (m, 6H), 6.20 (s, 1H), 5.97 (d, 1 H), 2.54 - 2.60 (m, 2H), 2.20 - 2.40 (m 1H), 1.80 - 2.00 (m, 1H), 1.35 - 1.85 (m, 8H), 0.95 - 1.35 (m, 6H). MS; pos. mode 390 (M + H).
Compound U. Name: 5-(2-benzofuran-2-yl-6-trifluoromethyl-4,5,6,7-tetrahydroindol-1-yl)-furan-2-carboxylic acid. 1H NMR, δ; CDCl3; 7.40 - 7.48 (m, 2H), 7.38 (d, 1H), 7.12 - 7.24 (m, 2H), 6.62 (s, 1H), 6.49 (d, 1H), 5.97 (s, 1H), 2.45 - 2.82 (m, 5H), 2.15 - 2.30 (m, 1H), 1.72 (qd, 1H). MS; pos. mode 416 (M + H).
Compound V. Name 5-(2-phenyl-6-trifluoromethyl-4,5,6,7-tetrahydroindol-1-yl)-furan-2-carboxylic acid. 1H NMR, δ; CDCl3; 7.19 - 7.32 (m, 4H), 7.05 - 7.18 (m, 2H), 6.22 (s, 1H), 6.07 (d, 1H), 2.45 - 2.80 (m, 5H), 2.20 (br d, 1H), 1.72 (qd, 1H). MS; pos. mode 376 (M + H).
Compound W. Name 5-(2-benzofuran-2-yl-5-trifluoromethyl-4,5,6,7-tetrahydroindol-1-yl)-furan-2-carboxylic acid. 1H NMR, δ; CDCl3; 7.36 - 7.47 (m, 3H), 7.13 - 7.24 (m, 2H), 6.63 (s, 1H), 6.45 (d, 1H), 6.00 (d, 1H), 2.86 (dd, 1H), 2.55 - 2.70 (m, 3H), 2.40 - 2.55 (br s, 1H), 2.23 (br d, 1H), 1.70 - 1.85 (m, 1H). MS; pos. mode 416 (M + H).
Compound X. Name: 5-[2-benzofuran-2-yl-5-(1,1-dimethylpropyl)-4,5,6,7-tetrahydroindol-1-yl]-furan-2-carboxylic acid. 1H NMR, δ; CDCl3; 7.35 - 7.44 (m, 3H), 7.12 - 7.22 (m, 2H), 6.60 (s, 1H), 6.41 (d, 1H), 5.97 (d, 1H), 2.58 (dd, 1H), 2.51 (br d, 2H), 2.27 - 2.35 (m, 1H), 1.99 (br d, 1H), 1.58 (td, 1H), 1.25 - 1.47 (m, 3 H), 0.90 (s, 3H), 0.89 (s, 3H), 0.84 (t, 3H). MS; pos. mode 418 (M + H).
Compound Y. Name: 5-(6-methyl-2-phenyl-4,5,6,7-tetrahydroindol-1-yl)furan-2-carboxylic acid. 1H NMR, δ; CDCl3: 7.1 - 7.3 (m, 6H); 6.2 (s, 1H); 6.0 (d, 1H); 2.5 - 2.6 (m, 3H); 2.2 (m, 1H); 1.9 (m, 2H); 1.4 (m, 1H); 1.1 (d, 3H). MS; pos. mode 322 (M + H), TOF EM 322.1449 (M + H).
Compound Z. Name: 5-[5-tButyl-2-(3,4-dichlorophenyl)-4,5,6,7-tetrahydroindol-1-yl] furan-2-carboxylic acid. 1H NMR, δ; CDCl3: 7.3 (m, 2H); 6.9 (dd, 2H); 6.2 (s, 1H); 6.0 (d, 1H); 2.5 - 2.6 (m, 4H); 2.3 (m, 1H); 2.0 (m, 1H); 1.4 (m, 1H); 1.0 (s, 9H). MS; pos. mode 432 (M + H); TOF EM 432.1122 (M + H).
Compound AA. Name: 5-[5-tButyl-2-(4-trifluoromethylphenyl)-4,5,6,7-tetrahydroindol-1-yl] furan-2-carboxylic acid. 1H NMR, δ; CDCl3: 7.5 (d, 2H); 7.1 - 7.3 (m, 3H); 6.3 (s, 1H); 6.0 (d, 1H); 2.5 - 2.6 (m, 3H); 2.3 (m, 1H); 2.0 (m, 1H); 1.4 (m, 1H); 1.0 (s, 9H). MS; TOF pos. mode 432 (M + H); EM 432.1783 (M + H).
Compound AB. Name: 5-(5-methyl-2-phenyl-4,5,6,7-tetrahydroindol-1-yl)furan-2-carboxylic acid. 1H NMR, δ; CDCl3: 7.1 - 7.3 (m, 6H); 6.1 (s, 1H); 6.0 (d, 1H); 2.5 - 2.7 (m, 3H); 2.1 (m, 1H); 1.9 (m, 2H); 1.4 (m, 1H); 1.0 (d, 3H). MS; pos. mode 322 (M + H); TOF EM 322.1476 (M + H).
Compound AC. Name: 2-(5-methyl-2-phenyl-4,5,6,7-tetrahydroindol-1-yl) thiazole-4-carboxylic acid. 1H NMR, δ; CDCl3: 8.0 (s, 1H); 7.2 - 7.3 (m, 5H); 6.2 (s, 1H); 2.7 (m, 2H); 2.6 (m, 1H); 2.2 (m, 1H); 1.9 (m, 2H); 1.5 (m, 1H); 1.0 (d, 3H). MS; TOF pos. mode 339 (M + H); EM 339.1175 (M + H).
Compound AD. Name 5-[2-(3,4-dichlorophenyl)-4-trifluoromethyl-4,5,6,7-tetrahydroindol-1-yl]-furan-2-carboxylic acid. 1H NMR, δ; CDCl3; 7.26 - 7.34 (m, 3H), 6.90 (dd, 1H), 6.43 (s, 1H), 6.17 (d, 1H), 3.40 - 3.50 (m, 1H), 2.45 - 2.65 (m, 2H), 1.95 - 2.15 (m, 2H), 1.70 - 1.95 (m, 2H).. MS; pos. mode 444 (M + H).
Compound AE. Name 5-[2-(3,4-dichlorophenyl)-6-trifluoromethyl-4,5,6,7-tetrahydroindol-1-yl]-furan-2-carboxylic acid. 1H NMR, δ; DMSO-d6; 13.4 (br s, 1H), 7.54 (d, 1H), 7.30 - 7.38 (m, 2H), 7.02 (dd, 1H), 6.73 (d, 1H), 6.56 (s, 1H), 2.81 (br s, 1H), 2.45 - 2.70 (m, 4H), 2.05 - 2.15 (m, 1H), 1.63 (qd, 1H). MS; pos. mode 444 (M + H).
Compound AF. Name 5-tButyl-2-phenyl-1-(4H-[1,2,4]triazol-3-yl)-4,5,6,7-tetrahydro-1H-indole. 1H NMR, δ; CDCl3: 8.1 (br. s., 1H); 8.0 (m, 0.4H); 7.7 (tt, 0.2H); 7.5 (tt, 0.3H); 7.1 - 7.2 (m, 5H); 6.2 (s, 1H); 2.5 - 2.6 (m, 3H); 2.3 (m, 1H); 2.0 (m, 1H); 1.4 - 1.5 (m, 2H); 1.0 (d, 9H). MS; pos. mode 321 (M + H); neg. mode 319 (M - H).
Compound AG. Name: 2-[2-(2,4-dimethoxyphenyl)-5-trifluoromethyl-4,5,6,7-tetrahydroindol-1-yl]-thiazole-4-carboxylic acid. 1H NMR, δ; DMSO-d6; 7.27 (s, 1H), 6.87 (d, 1H), 6.68 (m, 2H), 6.25 (s, 1H), 3.7 (s, 3H), 3.6 (s, 3H), 2.79 - 2.55 (m, 5H), 2.36 (s, 1H), 2.12 (d, 1H). MS; neg mode 452 (M dot).
Compound AH. Name: 2-[2-(3,4-dimethoxyphenyl)-5-trifluoromethyl-4,5,6,7-tetrahydroindol-1-yl]-thiazole-4-carboxylic acid. 1H NMR, δ; CDCl3; 7.18 (d, 1H), 6.54 (d, 1H); 6.48 (dd, 1H), 6.35 (d, 1H), 6.1 (s, 1H), 3.82 (s, 3H), 3.5 (s, 3H), 2.96 - 2.8 (m, 4H), 2.68 - 2.60 (m, 1H), 2.24 - 2.18 (m, 1H), 1.8 - 1.68 (m, 1H). MS; pos. mode 423 (M + H - OMe).
Compound AI. Name {2-[2-(3-chlorophenyl)-5-(1,1-dimethylpropyl)-4,5,6,7-tetrahydroindol-1-yl]-thiazol-4-yl}acetic acid. 1H NMR, δ; CDCl3; 7.10 - 7.22 (m, 3H), 7.02 (s, 1H), 6.60 - 7.01 (m, 1H), 6.24 (s, 1H), 3.83 (s, 2H), 2.60 - 2.70 (m, 2H), 2.54 (dd, 1H), 2.20 - 2.38 (m, 1H), 1.96 - 2.08 (m, 1H), 1.55 - 1.65 (m, 1H), 1.32 - 1.46 (m, 3H), 0.89 (s, 3H), 0.88 (s, 3H), 0.84 (t, 3H). MS; pos. mode 443 (M + 1).

### Validation of Methods of Prevention and Treatment

The disclosure herein addresses the ability of the disclosed compounds to correct axonal dysfunction in motor neurons, which is critical in a number of diseases and disorders affecting such neurons. Methods of treating diseases and disorders involving compromised axonal function are presented herein, to correct essentially any "downstream" dysfunction regardless of the source of the of "upstream" defect that leads to the compromised axonal function. While not wishing to be bound by theory, it is believed that the compounds of Formula I act *in vivo* to treat and/or prevent certain pathologies by correcting axonal dysfunction by specifically modulating a biochemical pathway associated with a vesicular transport pathway (e.g., axonal transport).

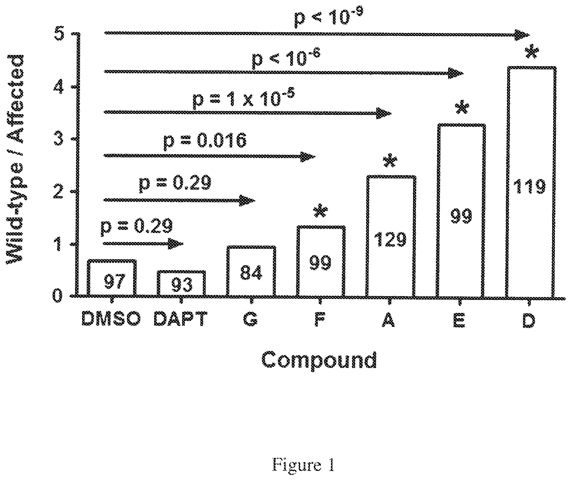

To evaluate this possibility, three classes of compounds were tested for rescue of locomotion of *khc*/+*; klc*/+ larvae, which lack one copy of the genes encoding the heavy and light chains of kinesin-1. One class of tested compounds consists of various exemplary compounds claimed herein, specifically compounds A, D, E and F (the structures and chemical names of which are shown in Table 1), each of which has been disclosed to lower Aβ42 production (U.S. Patent 7,678,823). The second class consists of one compound, Compound G, which is chemically similar to the exemplary compounds A, D, E and F, but fails to lower Aβ42 production. The third class consists of the compound DAPT (N-[N-(3,5-difluorophenacetyl-L-alanyl)]-S-phenylglycine t-butyl ester), which is chemically dissimilar to the exemplary compounds and which inhibits production not only of Aβ42, but of all other products of γ-secretase (Qi-Takahara et al, J Neurosci 2005, 25(2):436-445; Dovey et al, J Neurochem 2001, 76(1):173-181; Zhao et al, J Neurochem 2007, 100(5):1234-1246). Each of the exemplary compounds described herein, and previously disclosed to lower Aβ42 production, rescued locomotion of the mutant larvae (FIG. 1). In contrast, neither Compound G nor DAPT significantly affected the fraction of mutant larvae with motor dysfunction (FIG. 1). The lack of effect of DAPT cannot be explained in terms of inadequate concentrations of the compound, since the tested concentration has previously been reported to alter Notch-dependent phenotypes in *Drosophila* (Zhou et al, Proc Natl Acad Sci USA 2011, 108(6):2349-2354; Micchelli et al, FASEB J 2003, 17(1):79-81).

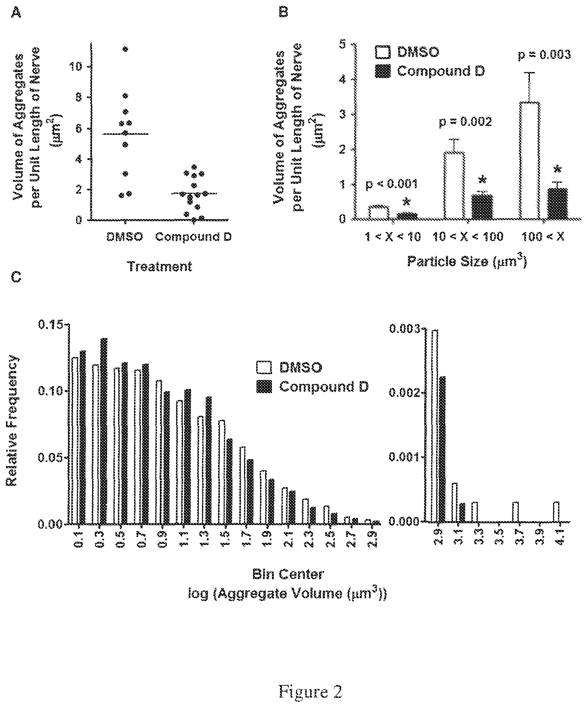

The aberrant locomotion of kinesin mutants is highly correlated with the accumulation in axons of membranous debris derived from vesicles, mitochondria, synaptic membranes, and pre-lysosomal organelles (Hurd 1996; Horiuchi et al, Curr Biol 2005, 15(23):2137-2141). Compounds that suppress the locomotion defect may also suppress the appearance of these membranous aggregates. Treatment of larvae with exemplary Compound D reduced the total volume of axonal aggregates to 30% of the level seen in vehicle-treated controls (FIG.2A). Reduction of the mass of membranous accumulations was not restricted to a particular size of aggregate. Thus, when aggregates were classified as small (1 µ³ < volume < 10 µ³), medium (10 µ³ < volume < 100 µ³), or large (100 µ³ < volume), reductions were observed for all three classes (FIG. 2B). This effect of Compound D is also indicated by a compound-induced shift in the frequency distribution of aggregate sizes towards smaller values (FIG. 2C).

The high levels of compounds in the medium on which larvae are raised (*viz.,* 0.5 mM) are typical of *Drosophila* studies that explore drug effects (Micchelli 2003; Steffan et al, Nature 2001, 413(6857):739-743; Ferrante et al, J Neurosci 2003, 23(28):9418-9427; Pollitt et al, Neuron 2003, 40(4):685-694; Moore et al, Cell 1998, 93(6):997-1007; Kang et al, Proc Natl Acad Sci USA 2002, 99(2):838-843; Pendleton et al, J Pharmacol Exp Ther 2002, 300(1):91-96; Mudher et al, Mol Psychiatry 2004, 9(5):522-530; Morfini et al, EMBO J 2002, 21(3):281-293). Still, it is possible that the levels of compounds used in the experiments result in very high concentrations in the animals. To address this possibility, the levels of Compound D in hemolymph of larvae raised on media containing 0.5 mM compound were assayed by liquid chromatography-tandem mass spectrometry (LC-MS/MS). This concentration of Compound D suppressed motor dysfunction in 69% of *khc*/+*; klc*/+ larvae (FIG. 1) and reduced by 70% the mass of membranous aggregates in their segmental nerves (FIG. 2A). No trace of Compound D was observed in the hemolymph of larvae treated with DMSO vehicle, indicating negligible background signal in the LC-MS/MS assay. The concentration of Compound D in the hemolymph of compound-treated larvae averaged 228 nM; concentrations in the independently collected duplicate samples were 207 and 248 nM. That is, the concentration in hemolymph was less than 1/2,000^{th} the nominal concentration in the media. There existed an apparent metabolite of Compound D of greater molecular weight, suggesting modification (*e.g.,* oxidation) of the parent compound. The signal from this single metabolite was equivalent to Compound D. Thus, the suppressor effects of Compound D and, by inference, its analogs, are determined to be observed at pharmacologically reasonable concentrations of compounds.

Compound D is one of several exemplary compounds that were tested for suppression of kinesin deficiency in *Drosophila* larvae, all of which showed positive effects (FIG 1), suggesting that the compounds affect anterograde transport in motor axons. Since extension and maintenance of axons and their growth cones depend on anterograde transport of membrane-bound organelles, protein complexes, and mRNA-containing particles (Mochida Neurosci Res 2011, 70(1):16-23; Goldstein et al, Curr Opin Neurobiol 2008, 18(5):495-503), these exemplary compounds may promote neurite outgrowth of motor neurons. This was tested using motor neurons isolated from embryonic rat spinal cords.

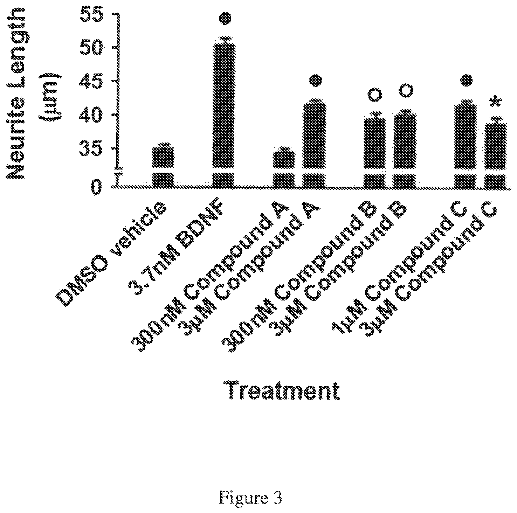

Of the exemplary compounds that were shown to suppress the locomotion defect of kinesin-deficient *Drosophila* larvae, Compound A exhibited superior PK (pharmacokinetic) and ADMET (absorption, distribution, excretion, metabolism, toxicity) properties in rats and mice. Because of its attractive PK and ADMET properties, Compound A was further evaluated by examining the compound's effects on neurite outgrowth of rat spinal motor neurons. Two additional exemplary compounds that likewise exhibit attractive PK and ADMET properties, Compounds B and C, were tested alongside Compound A. Motor neurons isolated from spinal cords of E15 rat embryos were cultured for 3 days in the presence of the three exemplary compounds and morphometrically analyzed to quantify neurite lengths. All three compounds significantly stimulated axon outgrowth from the isolated motor neurons (FIG. 3); that is, the compounds exhibited axonotrophic activity.

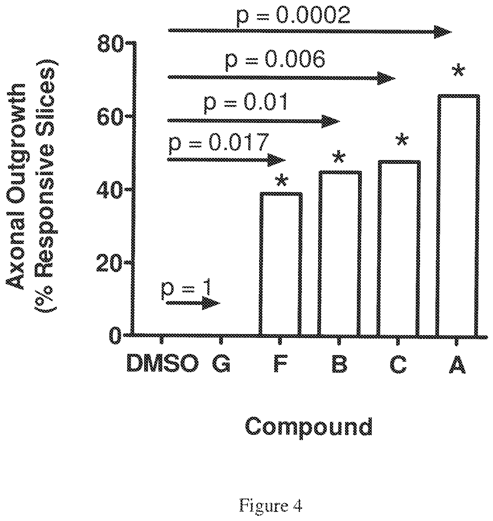

To evaluate the axonotrophic activity of the compounds on motor neurons in a more native biological context, organotypic spinal cord slices were treated with the same three exemplary compounds. Specifically, transverse sections of 8-day-old rat spinal cords were exposed to: exemplary Compounds A, B, or C; Compound G, which does not lower Aβ42 production or rescue locomotion of kinesin-deficient Drosophila larvae (FIG1); or DMSO vehicle. At least 15 individual spinal cord slices were treated with each compound. While no compound affected all slices treated with the compound, the exemplary compounds stimulated motor neuron outgrowth in a fraction of the treated slices (FIG. 4). In contrast, the DMSO vehicle and Compound G failed to affect any slice. Thus, each of the exemplary compounds exhibited detectable axonotrophic activity on motor neurons that reside in their native tissue environment. This result is particularly compelling given the inhibitory effects of the myelin component of spinal cord tissue on outgrowth of motor axons (Hannila and Filbin, Exp Neurol 2008 209(2):321-32).

SMA is caused by low levels of SMN protein (Monani, 2005), which can be modeled in zebrafish using antisense morpholino oligonucleotide (AMO) to decrease expression of Smn, the zebrafish homolog of human SMN protein (McWhorter et al, J Cell Biol 2003, 162(5):919-931). AMO-mediated reduction of Smn levels in zebrafish embryos results in motor axon defects such as truncation and aberrant branching, defects that are rescued by heterologous expression of human SMN. Such results indicate that the motor axon defects are indicative of low Smn levels and suggest that motor neuron development is abnormal when Smn levels are low (McWhorter 2003; Carrel et al, J Neurosci 2006, 26(43):11014-11022; Beattie et al, J Child Neurol 2007, 22(8):995-1003). To evaluate the effects of the exemplary compounds on motor axon development in Smn-deficient zebrafish embryos, a known scoring algorithm was applied, in which individual embryos are classified according to the number of defective motor axons and the severity of the defects (Carrel 2006). Thus, an embryo is classified as severe, moderate, mild, or unaffected on the basis of its motor axon defects (Akten 2011).

The Smn-deficient zebrafish is an important model for not just SMA, but also any motor neuron disease that involves compromised axonal function. These diseases include SMA, CMT2, HSP, hereditary sensory motor neuropathy and MS.

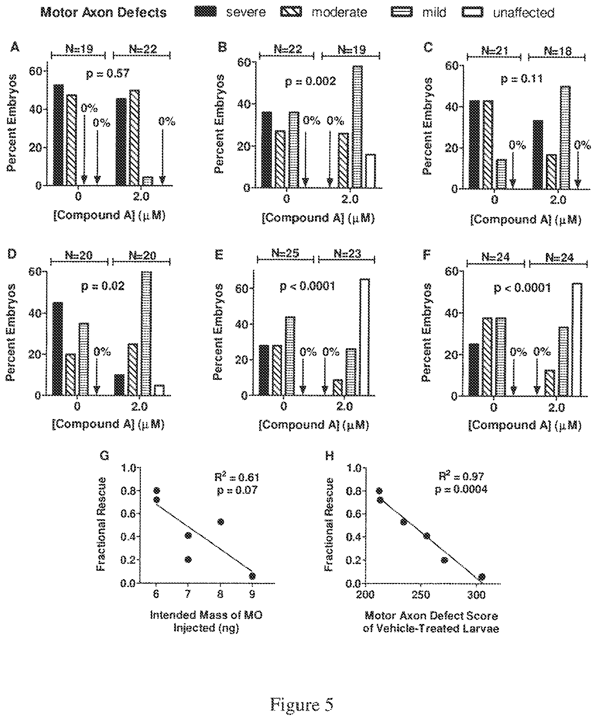

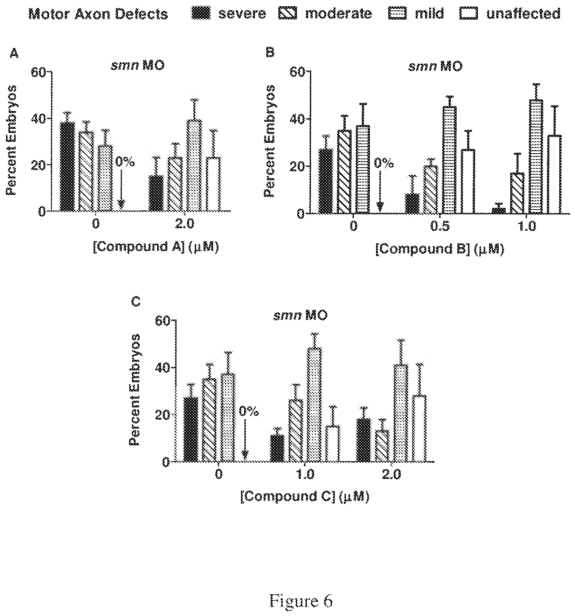

The ability of exemplary Compound A to rescue normal development of motor axons in Smn-deficient embryos is illustrated in FIGs 5 and 6. FIGs. 5A-F show results of the 6 experiments in which embryos were injected with Smn-reducing antisense morpholino (designated *smn* AMO) and allowed to develop in fish water containing either DMSO vehicle or Compound A from 10 to 28 hours post fertilization (hpf). In 4 of the 6 experiments, exposure of embryos to 2 µM Compound A resulted in a statistically significant redistribution of embryos among the severe, moderate, mild, or unaffected classes. Results averaged from the six experiments indicate that the compound significantly rescued the normal development of motor axons (FIG. 6A). The compound's effects are highlighted by considering embryos that were unaffected by Smn knockdown: among the 6 experiments, none of the 131 embryos exposed to DMSO vehicle were completely free of motor axon defects; in contrast 25% of compound-treated embryos (32 of 126) were unaffected.

To look at these data in more detail, a metric, Motor Axon Defect Score, was constructed to quantify the severity of motor axon defects. Motor Axon Defect Scores, in turn, were used to calculate the effectiveness of candidate suppressors, which were expressed as Fractional Rescue. A Fractional Rescue of 1 indicates the absence of any motor axon defects among compound-treated Smn-deficient embryos (that is, full rescue); a Fractional Rescue of 0 indicates equal motor axon defects between vehicle-treated and compound-treated Smn-deficient embryos (that is, no rescue). The Fractional Rescue was used to determine whether rescue was correlated with the dose of AMO or with the severity of the motor axon defects. The nominal amount of *smn* AMO injected into embryos varied from 6 to 9 ng among the experiments of FIGs. 5A-F. FIG. 5G plots the Fractional Rescue for Compound A as a function of the amount of *smn* AMO that was delivered in each experiment. Although there is a suggestion of an inverse correlation between the suppressor effect of Compound A and the nominal amount of *smn* AMO delivered, the slope of the linear regression is not significantly different from zero (p = 0.07). Several factors, such as aging-dependent changes in AMO concentration and differing efficacies among batches of AMO, could obscure a relationship between suppression and the amount of AMO delivered. A stronger association exists between the suppressor effect of Compound A and the Motor Axon Defect Score of vehicle-treated embryos (FIG. 5H). This analysis indicates that 97% of the variance in the suppressor effect of Compound A among the experiments in FIG. 5A-F can be attributed to various severities of *smn* AMO-induced motor axon defects. Importantly, the analysis also indicates that the greater the severity of the *smn* AMO-induced motor axon defects, the less effective the compound is in suppressing those defects.

Exemplary compounds B and C, which are analogs of Compound A that likewise show axonotrophic activities on spinal motor neurons (FIGs. 3 and 4), were also tested for rescue of Smn knockdown. Compound C was tested at both 1 and 2 µM; Compound B, because of its lower maximum nontoxic dose in zebrafish, was tested at 0.5 and 1 µM. As shown in FIGs. 6B-C, both compounds rescued motor axon development. Again, the suppressor effects of the compounds are illustrated by considering the AMO-injected embryos that were completely free of motor axon defects: no such unaffected embryos were among the 99 embryos exposed to DMSO vehicle; in contrast 30% of embryos treated with 1 µM Compound B (29 of 95) and 31% of embryos treated with 2 µM Compound C (27 of 88) were free of motor axon defects. Taken together, these data show that all three exemplary compounds alleviate the motor axon defects caused by low levels of Smn.

The exemplary compounds that rescued motor axon development in the zebrafish SMA model are modifiers of Aβ metabolism (U.S. Patent 7,678,823). One likely target of the compounds is γ-secretase, the enzyme complex responsible for production of Aβ peptides by proteolysis of APP. Since Notch, a protein critical for tissue development and homeostasis (Penton et al, Semin Cell Dev Biol 2012, 23(4):450-457), is another substrate of γ-secretase, compounds that target γ-secretase can have teratogenic or carcinogenic effects (Barten et al, Drugs R D 2006, 7(2):87-97). For this reason, developers of pharmaceuticals for Alzheimer's disease have expended efforts to identify compounds that modify γ-secretase-mediated processing of APP without affecting the enzyme's processing of Notch (Bulic et al, Curr Neuropharmacol 2011, 9(4):598-622). The pediatric nature of SMA magnifies concerns of the exemplary compounds' potential for detrimental effects on development.

The formation of somite borders in zebrafish larvae requires proper processing of Notch by γ-secretase, and the regularity of these somite boundaries provides a sensitive, *in vivo* indicator of accurate Notch processing (Holley Dev Dyn 2007, 236(6):1422-1449; Julich et al, Dev Biol 2005, 286(2):391-404; Jiang et al, Nature 2000, 408(6811):475-479; Gossler et al, Curr Top Dev Biol 1998, 38:225-287). At the concentrations that rescued motor axon development in Smn-deficient embryos, no effect of Compounds A, B and C on the somite borders of normal larvae was found. Indeed, irregular somite boundaries were never observed at concentrations below their toxic thresholds, which are at least twice the concentrations tested on Smn-deficient embryos. In contrast, treatment with 32µM DAPT, which has been shown to alter Notch processing in zebrafish (Geling et al, EMBO Rep 2002, 3(7):688-694), consistently disrupted the regularity of somite boundaries. Thus, the exemplary compounds' rescue of motor axon development is not associated with altered Notch processing.

Agents that discriminately modify the processing of APP by γ-secretase without affecting its activity on Notch have been designated γ-secretase modulators (Wolfe J Neurochem 2012, 120 Suppl 1:89-98). Thus, γ-secretase modulators are pharmacologically distinct from conventional γ-secretase inhibitors such as DAPT, which affect processing of all γ-secretase substrates. If Compounds A, B and C were γ-secretase modulators, it would reconcile the compounds' inactivity on Notch processing in zebrafish with their disclosed effects on Aβ metabolism (U.S. Patent 7,678,823). It would also rationalize the results shown in FIG. 1, where the compounds and DAPT have qualitatively different effects on locomotion of kinesin-deficient *Drosophila* larvae. In summary, these observations are consistent with the exemplary suppressor compounds behaving as γ-secretase modulators.

These results collectively indicate that the exemplary compounds promote the growth and function of motor axons. Of specific relevance to motor neuropathies, three of the exemplary compounds rescued development of motor axons in a zebrafish model of SMA. These findings suggest that the exemplary compounds may be useful as candidate therapeutics and axonotrophic agents for the treatment of any neuropathy involving compromised axonal function, including SMA, CMT2, HSP, hereditary sensory motor neuropathy and MS.

*Drosophila* larvae that are deficient in functional kinesin are relevant to human motor neuropathies (Djagaeva et al, Genetics 2012 192(1):173-83). Mutations in *Kif5A,* the human ortholog of *Drosophila Khc,* can cause both CMT2 and the SPG10 form of HSP (Blair et al, Neurogenetics 2006, 7(1):47-50; Crimella et al, Clin Genet 2012, 82(2):157-164; Fichera et al, Neurology 2004, 63(6):1108-1110; Reid et al, Am J Hum Genet 2002, 71(5):1189-1194; Salinas et al, Lancet Neurol 2008, 7(12):1127-1138). Like kinesin-deficient *Drosophila,* patients who suffer from CMT2 or the SPG10 form of HSP exhibit dystrophic axon termini, reduced axonal transport, and accumulation of axonal aggregates, all of which precede distal neuropathy (Hurd 1996; Salinas 2008; Saxton et al, Cell 1991, 64(6):1093-1102; Gho et al, Science 1992, 258(5080):313-316; Tarrade et al, Hum Mol Genet 2006, 15(24):3544-3558). Clearly, kinesin-deficient *Drosophila* models these diseases.

The relevance of kinesin-deficient *Drosophila* to motor neuropathies extends beyond diseases caused by mutant kinesin to other diseases in which axonal transport is compromised and axonal swellings are observed. These diseases include ALS (Delisle et al, J Neurol Sci 1984, 63(2):241-250; De Vos et al, Hum Mol Genet 2007, 16(22):2720-2728; Takahashi et al, Acta Neuropathol 1997, 94(3):294-299; Rao et al, Neurochem Res 2003, 28(7):1041-1047; Okamoto et al, Acta Neuropathol 1990, 80(2):222-226; Magrané et al, Antioxid Redox Signal. 2009, 11(7):1615-26; Perlson et al, Trends Neurosci 2010, 33(7):335-44), Huntington's disease (Morfini et al, Nat Neurosci 2009, 12(7):864-871; Gunawardena et al, Arch Neurol 2005, 62(1):46-51; Trushina et al, Mol Cell Biol 2004, 24:8195-8209; Perlson 2010), Parkinson's disease (Chung et al, J Neurosci 2009, 29(11):3365-3373; Mattila et al, Acta Neuropathol 1999, 98(2):157-164; Perlson 2010), forms of HSP in addition to SPG10 (Tarrade 2006; Kasher et al, J Neurochem 2009, 110(1):34-44; Salinas 2008), SMA (Dale et al, Acta Neuropathol 2011, 122(3):331-341; McGovern et al, Hum Mol Genet 2008, 17(18):2900-2909; Martinez-Hernandez et al, J Pathol 2012, 229(1):49-61; Rao 2003), SCA3 (Seidel et al, Acta Neuropathol 2010 120(4):449-60; Gunawardena 2005; Gunawardena et al, Neuron 2003 40(1):25-40; Feany and La Spada Neuron 2003 40(1):1-2), SCA6 (Takahashi et al, Neuropathology 2012 32(6):595-603; Ishiguro et al, Acta Neuropathol 2010 119(4):447-64; Seidel et al, Clin Neuropathol 2009 28(5):344-9; Ishikawa et al, Neurology 2001 56(12):1753-6), SCA5 (Dick et al, Handb Clin Neurol 2012, 103:451-9; Lorenzo et al, J Cell Biol 2010 189(1):143-58), SBMA (Katsuno et al, Prog Neurobiol 2012, 99(3):246-56; Kemp et al, Hum Mol Genet 2011, 20(22):4475-90; Kikuchi et al, Acta Neuropathol 2000, 99(1):63-6), dystonia musculorum (De Repentigny et al, PLoS One 2011, 6(6):e21093; Bernier et al, Dev Genet 1998, 22(2):160-8), FTDP-17 (Zhang et al, J Neurosci 2004 24(19):4657-67; Hong et al, Science 1998 282(5395):1914-7), MS (Ferguson et al, Brain 1997, 120(Pt 3):393-399; Anderson et al, Brain 2008, 131(Pt 7):1736-1748; Shriver and Dittel, Am J Pathol 2006, 169(3):999-1011), FTLD / FTD (Ghazi-Noori et al, Brain 2012, 135(Pt 3):819-832; Ittner et al, Proc Natl Acad Sci USA 2008 105(41):15997-6002; Martinaud et al, Acta Neuropathol 2005, 110(1):84-92), SCI (Beirowski et al, J Neuropathol Exp Neurol 2010, 69(5):455-72; Nashmi and Fehlings, Neuroscience 2001, 104(1):235-51), and Alzheimer's disease (Rao 2003; Dickson et al, Exp Neurol 1999, 156(1):100-110; Stokin et al, Science 2005, 307(5713):1282-1288; Perlson 2010). It is notable that, while Alzheimer's disease, multiple sclerosis, and frontotemporal lobar degeneration are not conventionally classified as motor neuropathies, they often have some degree of motor involvement. Such considerations emphasize the relevance of kinesin-deficient *Drosophila,* with their motor dysfunction, axonal swellings, and compromised axonal transport, to all of the diseases listed above.

Further, the axonotrophic characteristics of the exemplary compounds suggest they can be used as therapeutics for spinal cord injury (SCI). Particularly relevant to their potential as SCI therapeutics is ability of the compounds to stimulate growth of motor axons in spinal cord slices (Example 5 and Figure 4), where the presence of myelin normally inhibits axonal growth (Chaudhry et al, J Cereb Blood Flow Metab, 2007 27(6):1096-107). The inhibitory effect of myelin on axonal growth is a factor responsible for the inability of axons to regenerate after SCI. Without being bound by theory, the compounds herein disclosed appear to block the inhibitory effect of myelin on axonal growth. This characteristic, together with their ability to stimulate neurite outgrowth from isolated motor neurons (Example 4 and Figure 3), indicates their potential to promote recovery from the neurological damage of SCI.

Disclosed herein is evidence that certain of the exemplary compounds rescue normal locomotion of kinesin-deficient *Drosophila* larvae, promote neurite outgrowth of isolated spinal motor neurons, stimulate motor neuron growth in cultured spinal cord sections, and rescue motor axon development in Smn-deficient zebrafish. These findings have particular relevance for the development of SMA therapeutics and general relevance for the treatment of neuropathies that involve dysfunctional motor neurons, including SMA, CMT2, HSP, hereditary sensory motor neuropathy and MS.

### Example 3

Construction of *Drosophila* Strains: Reductions of kinesin function in *Drosophila* can result in a larval locomotion defect characterized by a rhythmic elevation of the tail due to paralysis of muscles in ventral posterior segments (Hurd et al, Genetics 1996, 142(1):195-204; Bowman et al, Cell 2000, 103(4):583-594). The phenotype is observed in animals with mutations in either the heavy or the light chains of kinesin-1 (formerly termed conventional kinesin), encoded by *Khc* and *Klc,* respectively. The severity and penetrance of the phenotype depends on the combination of mutant alleles carried by the larvae (Martin et al, Mol Biol Cell 1999, 10(11):3717-3728). Thus, *khc*/+*; klc*/+ double heterozygotes exhibit phenotypes that are intermediate between *khc*/*khc* or *klc*/*klc* homozygotes and single *khc* or *klc* heterozygotes. Furthermore, the severity of the phenotype of *khc*/+*; klc*/+ double heterozygotes depends on the particular alleles of *Khc* and *Klc.* A *khc*/+*; klc*/+ heterozygote was constructed that exhibits a penetrance of approximately 70%; it was presumed that the severity of the phenotype would be sufficiently low to be pharmacologically suppressible yet sufficiently high to allow discrimination of rescues with different efficacies. Such an intermediate penetrance also allows the formal possibility of identifying agents that exacerbate the locomotion defect. Thus, publicly available stocks of *b[1] pr[1] Khc[8]* / *CyO cy* and *y[1] w[*] ; T(2:3)B3 CyO ; TM6B Tb[1]* / *Pin[88K]* were used to obtain female *b[1] pr[1] Khc[8] ; T(2:3)B3 CyO ; TM6B Tb[1]* that were mated to male *w[*]* ; *Df(3L)8ex25* / *TM6B Tb[1]* animals. The female wild-type *Khc* allele can be followed using the larval marker Tubby to distinguish it from the amorphic *Khc[8]* (http://flybase.org). The male wild-type *Klc* allele, which encodes the kinesin light chain, is also marked by Tubby, allowing it to be distinguished from the deletion that includes the *Klc* locus. The *b[1] pr[1] Khc[8] ; Df(3L)8ex25* larval progeny from this cross, identified by their normal (non-tubby) body shape, are the experimental *khc*/+*; klc*/+ double heterozygotes that are scored herein. Fly stocks were crossed and maintained at 25°C; phenotypic assays were scored at room temperature.

Scoring of *Drosophila* Motor Phenotype: Experimental larvae were raised on Instant Drosophila Medium (Carolina Biological Supply, Burlington, NC) supplemented with 0.1% bromophenol blue and containing dimethyl sulfoxide (DMSO) alone or with test compounds dissolved in the DMSO vehicle. None of the test compounds had a discernible effect on larval development at the concentrations reported herein. The locomotion phenotype of non-tubby wandering third instar larvae was scored as either wild-type or uncoordinated, based on visual detection of the characteristic tail-flipping exhibited by kinesin-1 mutants (Hurd et al, Genetics 1996, 144(3):1075-1085). The person scoring the locomotion phenotype was unaware of the compound treatment of the animals.

As seen in FIG. 1, the results of *khc*/+*; klc*/+ larvae grown in the presence of vehicle alone (DMSO) or 0.5 mM of the indicated compounds were scored for motor dysfunction. The number of larvae scored for each treatment is indicated within the relevant bar. Results are expressed in terms of the number of larvae exhibiting no observable motor dysfunction (Wild-type) relative to the number with some degree of dysfunction (Affected). The extent of suppression of motor dysfunction is reflected by the heights of the bars relative to DMSO treatment. The Wild-type / Affected ratio for the vehicle condition reflects 60% penetrance of the locomotion defect. p values, calculated by application of Fisher's exact test to each experimental condition vs. DMSO, are shown. With the sequential Bonferroni method described by Holland (Psychological Bulletin 1988, 104(1):145-149) and Holm (Scand J Stat 1979, 6:65-67) to determine significance (*) at α = 0.05 (thereby accommodating multiple testing issues), the results indicate with 95% confidence that exemplary Compounds A, D, E and F rescue coordinated locomotion.

Measurements of Intraneuronal Aggregates: Immunostaining of segmental nerves of *Drosophila* larvae was performed as described (Hurd et al, Genetics 1996, 144(3):1075-1085). Experimental larvae were grown on Instant Drosophila Medium containing 0.5mM Compound D or DMSO vehicle until the wandering third instar stage, when they were dissected in calcium-free buffer (128mM NaCl, 2mM KCl, 1mM EGTA, 4mM MgCl₂, 5mM HEPES (pH 7.1)) and fixed in several changes of 4% paraformaldehyde. Larval pelts were then permeabilized with several washes of phosphate buffered saline (137mM NaCl, 2.7mM KCl, 10mM Na₂HPO₄, 2mM KH₂PO₄, pH7.4) containing 0.1% Triton X-100; stained overnight at 4°C with rabbit anti-synaptotagmin (Santa Cruz Biotechnology, Santa Cruz, CA); labeled with fluorescein (FITC)-conjugated anti-rabbit antibody (Molecular Probes, Eugene, OR); and mounted for confocal microscopy. Synaptotagmin labels synaptic vesicles, which can accumulate at sites within dysfunctional axons where membranous material is deposited to form structures that are variously termed axonal swellings, axonal spheroids, and neuritic beads. These synaptotagmin-marked, intraneuronal aggregates were imaged using FluoView® software on an Olympus FV1000 confocal laser scanning microscope (Olympus, Center Valley, PA). Images were taken from segmental nerves passing through larval segment A4 for standardization. A 60X oil immersion objective was used along with a 488nm excitation laser optimized for detection of the FITC fluorophore. Z-series stacked images were obtained at a step size of 05µm over a 10-30µm range for each field. Raw z-stacks were then processed using Volocity® 3D Image Analysis Software (v 2.5, Improvision (PerkinElmer) Waltham, MA) to render 3-D images and calculate the volumes of synaptotagmin-positive objects. Only individual, distinct swellings that were also larger than 1µm³ were processed.

FIG. 2(A) depicts the volume of aggregates per unit length of nerve plotted for *khc*/+*; klc*/+ larvae raised on media containing DMSO (N=10 larvae) or 0.5mM Compound D (N=14 larvae). Each data point represents one animal. The variance in the DMSO condition likely reflects incomplete penetrance (typical penetrance is about 0.7). Despite this variance, the mean values differ significantly (p < 0.001, unpaired t test).

FIG. 2(B) shows the mean volumes of aggregates per unit length of nerve for three classes of aggregate sizes in DMSO-treated (N=10; white bars) and Compound D-treated (N=14; black bars) larvae. Aggregate volumes per length of nerve are significantly (*) lower in Compound D-treated larvae regardless of size class (t test with Bonferroni method to determine significance (Holland 1988; Holm 1979).

FIG. 2(C) presents histograms of the volumes of the intraneuronal aggregates, with aggregates greater than 1µm³ distributed among 0.2 log unit-wide bins. The frequency distribution of the aggregates is shown for DMSO-treated (white bars; 3,359 objects) and Compound D-treated larvae (black bars; 3,551 objects). The differently scaled y-axis for bins containing the largest aggregates allows visual discrimination of histograms throughout the entire range of aggregate volumes. Exemplary Compound D significantly shifts the size distribution of aggregates to smaller volumes (Mann Whitney test; p < 0.001).

Measurements in Hemolymph: Wild-type *Drosophila* larvae were raised on media containing 0.5mM Compound D or DMSO (vehicle). Wandering third instar larvae that had recently (within 60 minutes) climbed from the media were collected, washed three times in phosphate buffered saline (PBS) containing 0.1% Tween 20 to remove compound that may adhere to the cuticle, and rinsed three times in PBS to remove both any remaining compound and residual Tween 20 from the washes. Washing steps consumed less than 20 minutes in total. Larvae were then dissected individually for collection of about 0.25µl hemolymph per animal. Since their food was stained with bromophenol blue, larvae could be dissected without damaging the clearly visible gut, thus avoiding contamination of collected hemolymph with intestinal contents. The visible presence of food in the gut indicated that a reservoir of compound- or vehicle-containing media existed within the gut throughout the procedure. Approximately 50µl of hemolymph were collected from experimental and from control larvae, diluted to 200µl in PBS, and stored at -20C until thawed for mass spectroscopic analysis. This was done with independent duplicate samples for both the experimental and control conditions, yielding a total of 4 samples that represented about 800 dissected larvae. Hemolymph samples were fortified with an internal standard, extracted with ethyl acetate, reconstituted, and subjected to liquid chromatography-tandem mass spectrometry (LC-MS/MS) analysis as follows. Extracted samples were injected onto a reversed phased liquid chromatography system (Shimadzu LC-10 with an Agilent Zorbax C-18, 50mm x 4.6mm column) serving an AB Sciex API4000 QTrap mass spectrometer. The mass spectrometer operated a multiple reaction monitoring method in positive ion mode with an electrospray ionization source. Synthetic standards were used to generate a calibration curve and quality control samples in a PBS surrogate matrix. The quantitation range was 1-1000ng/ml based on the analysis of 50µl of diluted hemolymph. Back-calculated values for each calibration standard and quality control sample were within 15% of the theoretical concentration, and the coefficients of determination for the calibration curves were > 0.99.

### Example 4

Neurite Outgrowth of Rat Spinal Motor Neurons. Pregnant Wistar rats were sacrificed at 15 days gestation by cervical dislocation, fetuses were removed, and fetal spinal cords were dissected into ice-cold medium of Leibovitz (L15, Gibco), where their meninges were carefully removed. The spinal cords were dissociated by treatment with trypsin (Gibco) for 30 minutes at 37°C in the presence of DNAse I (Boehringer Mannheim, France); proteolysis was terminated by addition of DMEM containing 10% fetal bovine serum (Gibco). The suspension was triturated using a 10ml pipette and a needle syringe followed by centrifugation at 580 x g for 10 min at room temperature (RT). The pellet of dissociated cells was resuspended in L15 medium, and the resulting suspension was centrifuged for 10 min at 180 x g at RT on a layer of 3.5% solution of bovine serum albumin in L15 medium. The supernatant was discarded, the pellet was resuspended in L15 supplemented with 1% DNAase I, the suspension was layered on a cushion of Optiprep® (Abcys, France), and the preparation was centrifuged at 400 x g for 25 min at RT. The upper phase, containing purified spinal motor neurons, was collected, resuspended in L15, and centrifuged at 800 x g for 10 min at RT. The cell pellet was finally resuspended in a defined culture medium consisting of Neurobasal Medium® (Gibco) supplemented with 2% B27® Supplements (Gibco) and 5mM L-glutamine (Gibco). Viable cells were counted in a Neubauer cytometer using the Trypan Blue exclusion test (Sigma-Aldrich). Approximately 30,000 purified rat spinal motor neurons were seeded on 35mm dishes (Nunc) coated with poly-L-lysine, allowed to adhere for 2 hours, and treated for three days with the compounds, brain-derived neurotrophic factor (BDNF) as positive control, or DMSO vehicle at 37°C in a humidified incubator with 5% CO₂-95% atmospheric air. The length of the longest unbranched neurite was determined for each of ∼80 neurons for each condition. The 13kd neurotrophin BDNF was tested at a concentration (3.7nM) that has near-maximal effects on neurite outgrowth.

FIG. 3 illustrates the stimulation of neurite outgrowth in rat spinal motor neurons in the presence of exemplary compounds. Primary cultures of rat embryonic spinal motor neurons were treated with BDNF at 3.7nM and with Compounds A, B and C at the two indicated concentrations of each compound. One-way ANOVA with Dunnett's post test indicates that the positive control BDNF and each compound enhanced neurite outgrowth relative to DMSO vehicle (N=3 cultures; ● p < 0.001; **o** p < 0.01; * p < 0.05).

### Example 5

Organotypic Spinal Cord Slices. Spinal cord organotypic slice cultures were prepared from 8-day-old Sprague Dawley rats. Animals were euthanized and spinal cords removed under sterile conditions and placed in Gey's balanced salt solution (Sigma-Aldrich). Spinal cords were cut into 250 µm sections using a McIlwain Tissue Chopper, and the freshly cut spinal cords were placed into tissue culture plate inserts (0.4 um pore size; 4.2 cm² filtration area; Millicell) inside 6 well plates pre-incubated with 1mL of organotypic growth media [50% MEM (Gibco), 25% Hanks balanced salt solution (Gibco), 25% heat-inactivated horse serum (Gibco), 0.8% Hepes Buffer Solution (Gibco) supplemented with penicillin and streptomycin]. Cultures were maintained for 1 week (with 2 media changes) prior to treatment. The treatment groups were: no treatment, control (DMSO vehicle), and compound alone at a final concentration of 100µM (10µL of 10mM stock into 1mL of media) over 10 days. At least 3 wells (15 spinal cord slices) were used for each treatment. Over the 10 day treatment period, cultures were replenished with fresh media and/or compound 3 times. After 10 days of treatment, cultures were fixed in 4% paraformaldehyde, washed in PBS, and immunostained with antibody SMI-31, which stains a phosphorylated epitope in neurofilaments H and M, in order to visualize motor neurons. Culture slices were assessed for the morphology of axons of motor neurons (specifically axon lengths). Slices that showed evidence of motor axon extension were scored as responsive to treatment.

The fraction of spinal cord slices that were responsive to each treatment is shown in FIG. 4. Exemplary Compounds A, B, C, and F were observed to stimulate the extension of motor axon bundles in about 40% to about 60% of spinal cord slices. Notably, the structural analog Compound G, which failed to rescue locomotion of kinesin-deficient Drosophila (FIG. 1) and which does not lower Aβ42 production, resembled DMSO in showing no enhanced extension of the motor neurons in treated spinal cord slices. p values, calculated by application of Fisher's exact test to each experimental condition vs. DMSO, are shown. The effects of Compounds A, B, C, and F are statistically significant (*) at α = 0.05 (Holland 1988).

### Example 6

Selection of Test Concentrations in Zebrafish (*Danio rerio*). Selection of concentrations of small molecules for treating *D. rerio Tg(mnx1:GFP)* embryos that had been injected with antisense morpholino oligonucleotides (AMO) was guided by determinations of their maximum nontoxic concentrations (MTDs) in wild-type AB/Tübingen embryos and larvae. These MTDs were determined by exposing embryos to compound dissolved in egg water containing 0.25% DMSO or to the DMSO vehicle alone beginning at 4, 11, and 25 hours post-fertilization (hpf) and continuing exposure for 7 days; media was replaced with fresh media every 48 hours after initiation of exposure. The following parameters were monitored throughout the treatment period: morphology (gross body shape), viability (strength of escape reflex), growth (larval length), somite boundaries (regularity of borders between somites), swim bladder development (inflation of swim bladder), and pigmentation (level of pigmentation of yolk sac and yolk sac extension). Observed MTDs were not noticeably affected by dechorionation of the embryos. Based on these observations in wild-type embryos and larvae, AMO-injected *Tg(mnx1:GFP)* embryos with intact chorions were treated for a total of 18 hours, from 10 to 28 hpf, with Compounds A and C at 2µM and with the slightly less tolerated Compound B at 1µM. Compounds B and C were also tested at 50% lower concentrations *(viz.,* 0.5 µM and 1µM, respectively). As expected from the toxicity testing in wild-type embryos, these concentrations appeared nontoxic to AMO-injected *Tg(mnx1:GFP)* embryos.

Zebrafish Motor Axon Morphology: Zebrafish and embryos were maintained at 28.5°C and staged by hours post-fertilization (hpf) (Westerfield, The Zebrafish Book. A Guide for the Laboratory Use of Zebrafish (Danio rerio), 3rd ed. Eugene, OR: University of Oregon Press; 1995). Transgenic *Tg(mnx1:0.6hsp70:GFP)os26* zebrafish embryos that express GFP in ventrally projecting motor axons (Dalgin et al, Development 2011, 138(21):4597-4608), referred to as *Tg(mnx1:GFP)* embryos, were used for all knockdown experiments. Specifically, using an MPPI-2 Pressure Injector (Applied Scientific Instrumentation, Eugene, OR) and according to previous protocols (Carrel 2006), *Tg(mnx1:GFP)* embryos were injected at the one- to two-cell stage with the AMO CGACATCTTCTGCACCATTGGC (Gene Tools, Philomath, OR) to knock down Smn as previously described (McWhorter 2003). At 10 hpf injected embryos were placed in egg water (60µg/ml Instant Ocean® sea salts) containing compound or DMSO (0.25%) vehicle and incubated at 28.5°C. To visualize motor axons in GFP transgenic animals, *Tg(mnx1:GFP)* embryos at 28 hpf were anesthetized with tricaine and fixed overnight at 4°C in 4% formaldehyde/PBS. After removing embryos from the fixative, their yolks and heads were removed and their trunks were mounted on glass coverslips for observation under a Zeiss Axioplan microscope. Motor axons innervating the mid-trunk (myotomes 6-15) on both sides of the fish were scored as described (Carrel 2006), which allowed each embryo to be classified as severe, moderate, mild, or unaffected according to previously described criteria based on the number and types of motor axon abnormalities (Akten 2011). The effectiveness of a compound in suppressing the aberrant motor axon morphologies is assessed as the compound's effect on the distribution of embryos among the severe, moderate, mild, or unaffected classes.

FIG. 5 shows the rescue of motor axon development in Smn-deficient zebrafish embryos by Compound A. In FIGs. 5A-F, zebrafish embyros that had been injected with Smn-reducing antisense morpholino (designated *smn* AMO) and treated with either DMSO vehicle or 2µM Compound A were classified by degree of motor axon abnormalities. Distributions of embryos among the 4 classes of severity of motor axon dysmorphism are shown for the vehicle and Compound A conditions for each of 6 experiments. The p values comparing the two distributions were calculated for the individual experiments by the Mann-Whitney U test and are indicated in each graph along with the numbers of vehicle- and compound-treated embryos scored in that experiment. For any single embryo, approximately 20 axons were evaluated, resulting in about 400 axons scored for each of the control and experimental conditions in each experiment. The nominal mass of *smn* AMO injected varied among experiments: Expt. A: 9ng; Expt.B: 8ng; Expt.C: 7ng; Expt.D: 7ng; Expt.E: 6ng; Expt.F: 6ng. In FIGs. 5G-H, suppression of motor axon abnormalities in embryos injected with *smn* AMO by 2µM Compound A is expressed in terms of Fractional Rescue, as described herein. The Fractional Rescues for the 6 individual experiments shown in FIGs. 5A-F are plotted as a function of two different measures of the amount of *smn* AMO delivered to the embryos: FIG. 5G shows the nominal, intended mass of injected AMO; FIG. 5H shows an amount of *smn* AMO reflected by the Motor Axon Defect score, a metric of the severity of motor axon abnormalities, of vehicle-treated embryos. Parameters of the linear regressions in each graph are indicated. The two experiments associated with the lowest Fractional Rescues (values of 0.2 and 0.06, corresponding to panels A and C, respectively) failed to show significant rescue of motor axon development by Compound A.

FIG. 6 illustrates the rescue of motor axon development by axonotrophic compounds. The results with 2µM Compound A were averaged across the experiments in FIGs. 5A-F for both the control (vehicle-treated embryos) and experimental conditions, and mean values are plotted along with SEM (N = 6) in FIG 6A. Compound A significantly reduced the severity of motor axon defects (p < 0.0001, N = 6; Mann-Whitney U test). FIG. 6B shows the suppression of Smn knockdown by Compound B, which was tested in 5 experiments, each of which examined 3 conditions: 0.5µM Compound B (95 embryos scored among the 5 experiments, with about 20 embryos per experiment), 1µM Compound B (95 embryos), and DMSO vehicle (99 embryos). Both concentrations of Compound B significantly reduce the severity of motor neuron dysmorphism (p < 0.0001, N = 5; Kruskal-Wallis one-way ANOVA). FIG. 6C shows the suppression of Smn knockdown by Compound C, which was tested in 5 experiments, each of which examined 3 conditions: 1µM Compound C (88 embryos), 2µM Compound C (88 embryos), and DMSO vehicle (99 embryos). Both concentrations of Compound C significantly reduce the severity of motor neuron dysmorphism (p < 0.0001, N = 5; Kruskal-Wallis one-way ANOVA).

The biological assays described in the examples above are relevant to any neuropathy characterized by compromised axonal function. The flipper assay, which uses a *Drosophila* mutant that is deficient in the motor protein kinesin, is relevant because kinesin activity is essential for the growth, maintenance, and physiological function of axons. The assay of motor neuron growth in spinal cord explants is relevant because the neuronal growth reflects axonal viability in the context of spinal cord tissue. The assay of neurite outgrowth of isolated motor neurons is relevant because the neurite outgrowth reflects the inherent, cell-autonomous viability of developing axons. The assay of motor axon growth in Smn-deficient zebrafish is relevant because these animals model not only SMA but any other neuropathy in which axonal function is compromised.

The assays described above thus provide data supporting the ability of the disclosed compounds to broadly correct axonal dysfunction regardless of the source of the dysfunction. Methods of treating any disease or disorder involving compromised axonal function are possible, since the "downstream" result (i.e. the axonal blockage or dysfunction) may be alleviated by treatment with the disclosed compounds, regardless of the particular "upstream" defect, including, for example, defective kinesin motor proteins, that leads to the compromised axonal function.

## Claims

1. A compound with the structure of Formula I: 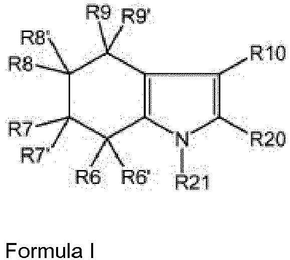
wherein R6-R10 and R6'-R9' are independently selected from at least one of hydrogen, hydroxyl, halo, alkyl, alkoxy, haloalkyl, haloalkoxy, -N(C₁₋₃ alkyl)2, -NH(C₁₋₃ alkyl), -C(=O)NH₂,-C(=O)NH(C₁₋₃ alkyl), -C(=O)N(C₁₋₃ alkyl)2, -S(=O)₂(C₁₋₃ alkyl), -S(=O)₂NH₂, -S(=O)₂N(C₁₋₃ alkyl)2, -S(=O)₂NH(C₁₋₃ alkyl), -CHF₂, -OCF₃, -OCHF₂, -SCF₃, -CF₃, -CN, -NH₂, and -NO₂;
R20 is optionally substituted aryl or heteroaryl; and
R21 is optionally substituted heteroaryl; or a pharmaceutically-acceptable salt thereof, for use in a method of treating a neurodegenerative disease or disorder associated with compromised axonal function selected from Charcot-Marie-Tooth Disease 2, spinal muscular atrophy, hereditary sensory motor neuropathy, hereditary spastic paraplegia, and multiple sclerosis in a human patient.

2. A compound for use according to claim 1, wherein R6 and R6' are hydrogen.

3. A compound for use according to claim 1 or 2 wherein R7, R7', R8, R8', R9 and R9' are independently selected from at least one of hydrogen, alkyl, haloalkyl, -CHF₂, -OCF₃,-OCHF₂, -SCF₃, -CF₃, -CN, -NH₂, and -NO₂.

4. A compound for use according to any preceding claim wherein R10 is hydrogen.

5. A compound for use according to any preceding claim wherein R20 is aryl or heteroaryl.

6. A compound for use according to claim 5 wherein R20 is phenyl or 3,4-dichlorophenyl.

7. A compound for use according to any preceding claim, wherein R7, R7', R8 and R8' are independently selected from at least one of hydrogen, alkyl and haloalkyl.

8. A compound for use according to any preceding claim, wherein R9 is alkyl or haloalkyl.

9. A compound for use according to any preceding claim, wherein R9' is hydrogen.

10. A compound for use according to any preceding claim, wherein R20 is aryl or benzofuranyl.

11. A compound for use according to any preceding claim, wherein R6, R6', R7, R7', R8, R9, R9' and R10 are hydrogen.

12. A compound for use according to any preceding claim, wherein R8' is 1,1-dimethylpropyl or trifluoromethyl.

13. A compound for use according to any preceding claim, wherein R21 is optionally substituted 5 or 6 membered heteroaryl.

14. A compound for use according to any preceding claim, wherein R21 is furan-2-carboxylic acid or thiazol-4-yl acetic acid.

15. A compound according to any one of the preceding claims for use according to any one of the preceding claims, wherein said neurodegenerative disease or disorder associated with compromised axonal function is spinal muscular atrophy.

16. A compound for use according to any one of the preceding claims, wherein the compound is selected from at least one of:
5-[5-(1,1-dimethyl-propyl)-2-phenyl-4,5,6,7-tetrahydro-indol-1-yl]-furan-2-carboxylic acid;
5- [2-(3,4-dichloro-phenyl)-5-trifluoromethyl-4,5,6,7-tetrahydro-indol-1-yl]-furan-2-carboxylic acid
{2-[5-(1,1-dimethylpropyl)-2-phenyl-4,5,6,7-tetrahydroindol-1-yl]-thiazol-4-yl} acetic acid;
6-(2-phenyl-4,5,6,7-tetrahydro-1H-indol-1-yl) picolinic acid;
4-(2-phenyl-4,5,6,7-tetrahydro-1H-indol-1-yl) picolinic acid;
5-(2-phenyl-4,5,6,7-tetrahydro-1H-indol-1-yl) nicotinic acid;
2-(2-phenyl-4,5 ,6,7-tetrahydro- 1H-indol-1-yl) isonicotinic acid;
4-(2-phenyl-4,5,6,7-tetrahydro-1H-indol-1-yl) nicotinic acid;
2-(2-phenyl-4,5,6,7-tetrahydro- 1H-indol-1-yl) pyrimidine-4-carboxylic acid;
5-(2-phenyl-4,5 ,6,7-tetrahydro- 1H-indol-1-yl)-1H-pyrrole-2-carboxylic acid;
4- (2-phenyl-4,5 ,6,7-tetrahydro-1H-indol-1-yl) furan-2-carboxylic acid;
5- (5-tert-butyl-2-phenyl-4,5,6,7-tetrahydroindol-1-yl) furan-2-carboxylic acid methyl ester;
5-(5-tert-butyl-2-phenyl-4,5,6,7-tetrahydroindol-1-yl) furan-2-carboxylic acid;
5-(2-phenyl-5-trifluoromethyl-4,5,6,7-tetrahydroindol-1-yl)-furan-2-carboxylic acid;
5-[2-(4-morpholin-4-yl-phenyl)-5-trifluoromethyl-4,5,6,7-tetrahydroindol-1-yl]-furan-2-carboxylic acid;
5-(5-cyclohexyl-2-phenyl-4,5,6,7-tetrahydroindol-1-yl)-furan-2-carboxylic acid;
5-(2-benzofuran-2-yl-6-trifluoromethyl-4,5,6,7-tetrahydroindol-1-yl)-furan-2-carboxylic acid;
5-(2-phenyl-6-trifluoromethyl-4,5,6,7-tetrahydroindol-1-yl)-furan-2-carboxylic acid;
5-(2-benzofuran-2-yl-5-trifluoromethyl-4,5,6,7-tetrahydroindol-l-yl)-furan-2-carboxylic acid;
5-[2-benzofuran-2-yl-5-(1, 1 -dimethylpropyl)-4,5,6,7-tetrahydroindol- 1 -yl]-furan-2-carboxylic acid;
5-(6-methyl-2-phenyl-4,5,6,7-tetrahydroindol-1-yl) furan-2-carboxylic acid;
5-[5-tert-butyl-2-(3,4-dichlorophenyl)-4,5,6,7-tetrahydroindol-1-yl] furan-2-carboxylic acid;
5-[5-tert-butyl-2-(4-trifluoromethylphenyl)-4,5,6,7-tetrahydroindol-1-yl]furan-2-carboxylic acid;
5-(5-methyl-2-phenyl-4,5,6,7-tetrahydroindol-1-yl) furan-2-carboxylic acid;
2-(5-methyl-2-phenyl-4,5,6,7-tetrahydroindol-1-yl) thiazole-4-carboxylic acid;
5-[2-(3,4-dichlorophenyl)-4-trifluoromethyl-4,5,6,7-tetrahydroindol-1-yl]-furan-2-carboxylic acid;
5-[2-(3,4-dichlorophenyl)-6-trifluoromethyl-4,5,6,7-tetrahydroindol-1-yl]-furan-2-carboxylic acid;
5-tert-butyl-2-phenyl-1-(4H-[1,2,4]triazol-3-yl)-4,5,6,7-tetrahydro-1H-indole;
2-[2-(2,4-dimethoxyphenyl)-5-trifluoromethyl-4,5,6,7-tetrahydroindol-1-yl]-thiazole-4-carboxylic acid;
2-[2-(3,4-dimethoxyphenyl)-5-trifluoromethyl-4,5,6,7-tetrahydroindol-1-yl]-thiazole-4-carboxylic acid;
{2-[2-(3-chlorophenyl)-5-(1,1-dimethylpropyl)-4,5,6,7-tetrahydroindol-1-yl]-thiazol-4-yl} acetic acid;
and pharmaceutically-acceptable salts of any of the foregoing.

## Patentansprüche

1. Verbindung mit der Struktur von Formel I: 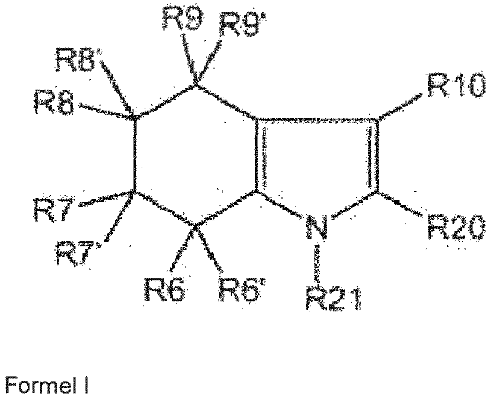 wobei
R6-R10 und R6'-R9' unabhängig ausgewählt werden aus mindestens einem von Hydrogen, Hydroxyl, Halogen, Alkyl, Alkoxy, Halogenalkoxy, -N(C₁₋₃ alkyl)₂, -NH(C₁₋₂ alkyl), -C(=O)NH₂, -C(=O)NH(C₁₋₃ alkyl), -C(=O)N(C₁₋₃ alkyl)₂, -S(=O)₂(C₁₋₃ alkyl),-S(O)₂NH₂, -S(=O)₂N(C₁₋₃ alkyl)₂, -S(=O)₂NH(C₁₋₃ alkyl), -CHF₂, -OCF₃, -SCF₃, -CF₃,-CN, -NH₂ und -NO₂;
R20 optional substituiertes Aryl oder Heteroaryl ist; und
R21 optional substituiertes Heteroaryl ist; oder ein pharmazeutisch akzeptables Salz davon, zur Verwendung in einem Verfahren zur Behandlung von neurodegenerativen Störungen oder Erkrankungen, die beeinträchtigter axonaler Funktion zugeordnet sind aus Charcot-Marie-Tooth-Srörung 2, spinaler Muskelatrophie, hereditärer motorisch-sensibler Neuropathie, hereditärer spastischer Paraplegie und multipler Sklerose in einem menschlichen Patienten.

2. Verbindung zur Verwendung nach Anspruch 1, wobei R6 und R6' Wasserstoff sind.

3. Verbindung zur Verwendung nach Anspruch 1 oder 2, wobei R7, R7', R8, R8', R9 und R9' unabhängig ausgewählt sind aus mindestens einem von Wasserstoff, Alkyl, Halogenalkyl, -CHF₂, -OCF₃, -OCHF₂, -SCF₃, -CF₃, -CN. -NH₂ und NO₂.

4. Verbindung zur Verwendung nach einem der vorhergehenden Ansprüche, wobei R10 Wasserstoff ist.

5. Verbindung zur Verwendung nach einem der vorhergehenden Ansprüche, wobei R20 Aryl oder Heteroaryl ist.

6. Verbindung zur Verwendung nach Anspruch 5, wobei R20 Phenyl oder 3,4-Dichlorphenyl ist.

7. Verbindung zur Verwendung nach einem der vorhergehenden Ansprüche, wobei R7, R7', R8 und R8' unabhängig ausgewählt sind aus mindestens einem von Wasserstoff, Alkyl und Halogenalkyl.

8. Verbindung zur Verwendung nach einem der vorhergehenden Ansprüche, wobei R9 Alkyl oder Halogenalky ist.

9. Verbindung zur Verwendung nach einem der vorhergehenden Ansprüche, wobei R9' Wasserstoff ist.

10. Verbindung zur Verwendung nach einem der vorhergehenden Ansprüche, wobei R20 Aryl oder Benzofuranyl ist.

11. Verbindung zur Verwendung nach einem der vorhergehenden Ansprüche, wobei R6, R6', R7, R7', R8, R9, R9' und R10 Wasserstoff sind.

12. Verbindung zur Verwendung nach einem der vorhergehenden Ansprüche, wobei R8' 1,1-Dimethylpropyl oder Trifluormethyl ist.

13. Verbindung zur Verwendung nach einem der vorhergehenden Ansprüche, wobei R21 optional substituiertes 5- oder 6-gliediriges Heteroaryl ist.

14. Verbindung zur Verwendung nach einem der vorhergehenden Ansprüche, wobei R21 Furan-2-Carbonsäure oder Thiazol-4-yl-Essigsäure ist.

15. Verbindung nach einem der vorhergehenden Ansprüche zur Verwendung nach einem der vorhergehenden Ansprüche, wobei die neurodegenerative Störung oder Erkrankung, die der beeinträchtigten axonalen Funktion zugeordnet wird, spinale Muskelatrophie ist.

16. Verbindung zur Verwendung nach einem der vorhergehenden Ansprüche, wobei die Verbindung ausgewählt wird aus mindestens einem aus:
5-[5-(1,1-Dimethyl-Propyl)-2-Phenyl-4,5,6,7-Tetrahydro-Indol-1-yl]-furan-2-Carbonsäure;
5-[2-(3,4-Dichioro-Phenyl)-5-Trifluormethyl-4,5,6,7-Tetrahydro-Indol-1-yl]-furan-2-Carbonsäure;
{2-[5-1(1,1-Dimethylpropyl)-2-Phenyl-4,5,6,7-Tetrahydroindol-1-yl]-Thiazol-4-yl}-Essigsäure;
6-(2-Phenyl-4,5,6,7-Tetrahydro-1H-Indol-1-yl)-Picolinsäure;
4-(2-Phenyl-4,5,6,7-Tetrahydro-1H-Indol-1-yl)-Picolinsäure;
5-(2-Phenyl-4,5,6,7-Tetrahydro-1H-lndol-1-yl)-Nikotinsäure;
2-(2-Phenyl-4,5,6,7-Tetrahydro-1H-Indol-1-yl)-Isonicotinsäure;
4-(2-Phenyl-4,5,6,7-Tetrahydro-1H-Indol-1-yl)-Nikotinsäure;
2-(2-Phenyl-4,5,6,7-Tetrahydro-1H-Indol-1-yl)-Pyrimidin-4-Carbonsäure;
5-(2-Phenyl-4,5,6,7-Tetrahydro-1H-Indol-1-yl)-furan-2-Carbonsäure;
5-(5-Tert-Butyl-2-Phenyl-4,5,6,7-Tetrahydroindol-1-yl)-Furan-2-CarbonsäureMethylester;
5-(5-Tert-Butyl-2-Phenyl-4,5,6,7-Tetrahydroindol-1-yl)-Furan-2-Carbonsäure;
5-(2-Phenyl-5-Trifluormethyl-4,5,6,7-Tetrahydroindol-1-yl)-Furan-2-Carbonsäure;
5-[2-(4-Morpholin-4-yl-Phenyl)-5-Trifluormethyl-4,5,6,7-Tetrahydroindol-1-yl]-Furan-2-Carbonsäure;
5-(5-Cyclohexyl-2-Phenyl-4,5,6,7-Tetrahydroindol-1-yl]-Furan-2-Carbonsäure;
5-(2-Benzofuran-2-yl-6-Trifluormethyl-4,5,6,7-Tetrahydroindol-1-yl)-Furan-2-Carbonsäure;
5-(2-Phenyl-6-Trifluormethyl-4,5,6,7-Tetrahydroindol-1-yl)-Furan-2-Carbonsäure;
5-(2-Benzofuran-2-y)-5-Trifluormethyl-4,5,6,7-Tetrahydroindol-1-yl)-Furan-2-Carbonsäure;
5-[2-Benzofuran-2-yl-5-(1,1-Dimethylpropyl)-4,5,6,7-Tetrahydroindol-1-yl]-Furan-2-Carbonsäure;
5-(6-Methyl-2-Phenyl-4,5,6,7-Tetrahydroindol-1-yl)-Furan-2-Carbonsäure;
5-[5-Tert-Butyl-2-(3,4-Dichlorphenyl)-4,5,6,7-Tetrahydroindol-1-yl)-Furan-2-Carbonsäure;
5-[5-Tert-Butyl-2-(4-Trifluormethylphenyl)-4,5,6,7-Tetrahydroindol-1-yl)-Furan-2-Carbonsäure;
5-(5-Methyl-2-Phenyl-4,5,6,7-Tetrahydroindol-1-yl)-Furan-2-Carbonsäure;
2-(5-Methyl-2-Phenyl-4,5,6,7-Tetrahydroindol-1-yl)-Thiazol-4-Carbonsäure;
5-[2-(3,4-Dichlorphenyl)-4-Trifluormethyl-4,5,6,7-Tetrahydroindol-1-yl)-Furan-2-Carbonsäure;
5-[2-(3,4-Dichlorphenyl)-6-Trifluormethyl-4,5,6,7-Tetrahydroindol-1-yl)-Furan-2-Carbonsäure;
5-Tert-Butyl-2-Phenyl-1-(4h-[1,2,4]-Triazol-3-yl)-4,5,6,7-Tetrahydro-1H-indole;
2-[2-(2,4-Dimethoxypheny))-5-Trifluormethyl-4,5,6,7-Tetrahydroindol-1-yl)-Thiazol-4-Carbonsäure;
2-[2-(3,4-Dimethoxyphenyl)-5-Thfluormethyl-4,5,6,7-Tetrahydroindol-1-yl)-Thiazol-4-Carbonsäure;
{2-[2-(3-Chlorophenyl)-5-(1,1-Dimethylpropyl)-4,5,6,7-Tetrahydroindol-1-yl]-Thiazol-4-yl}-Essigsäure;
und pharmazeutisch akzeptablen Salzen von einem der vorgehenden Stoffe.

## Revendications

1. Composé ayant une structure de formule I : 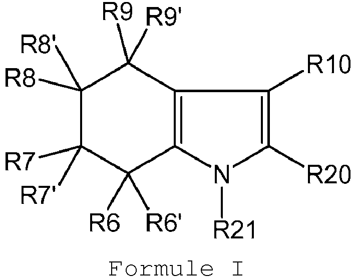
dans laquelle R6-R10 et R6'-R9' sont indépendamment au moins l'un choisi parmi l'hydrogène, hydroxyle, halogéno, alkyle, alcoxy, halogénoalkyle, halogénoalcoxy, -N(alkyle en C₁ à C₃)₂, -NH-alkyle en C₁ à C₃, -C(=O)NH₂, -C(=O)NH-alkyle en C₁ à C₃, -C(=O)N(alkyle en C₁ à C₃)₂, -S(=O)₂-alkyle en C₁ à C₃, -S(=O)₂NH₂, -S(=O)₂N (alkyle en C₁ à C₃)₂, -S(=O)₂NH-alkyle en C₁ à C₃, -CHF₂, -OCF₃, -OCHF₂, -SCF₃, -CF₃, -CN, -NH₂, et -NO₂ ;
R20 est un aryle ou hétéroaryle éventuellement substitué ; et
R21 est un hétéroaryle éventuellement substitué ;
ou un sel pharmaceutiquement acceptable de celui-ci, pour une utilisation dans une méthode de traitement d'une maladie ou d'un trouble neurodégénératif associé à une fonction axonale compromise choisi parmi la maladie de Charcot-Marie de type 2, l'amyotrophie spinale, la neuropathie sensitivomotrice héréditaire, la paraplégie spastique héréditaire, et la sclérose en plaques chez un patient humain.

2. Composé pour une utilisation selon la revendication 1, dans laquelle R6 et R6' sont des atomes d'hydrogène.

3. Composé pour une utilisation selon la revendication 1 ou 2, dans lequel R7, R7', R8, R8', R9 et R9' sont indépendamment au moins l'un choisi parmi l'hydrogène, alkyle, halogénoalkyle, -CHF₂, -OCF₃, -OCHF₂, -SCF₃, -CF₃, -CN, -NH₂, et -NO₂.

4. Composé pour une utilisation selon l'une quelconque des revendications précédentes, dans lequel R10 est l'hydrogène.

5. Composé pour une utilisation selon l'une quelconque des revendications précédentes, dans lequel R20 est un aryle ou hétéroaryle.

6. Composé pour une utilisation selon la revendication 5, dans lequel R20 est un phényle ou 3,4-dichlorophényle.

7. Composé pour une utilisation selon l'une quelconque des revendications précédentes, dans lequel R7, R7', R8 et R8' sont indépendamment au moins l'un choisi parmi l'hydrogène, alkyle et halogénoalkyle.

8. Composé pour une utilisation selon l'une quelconque des revendications précédentes, dans lequel R9 est un alkyle ou halogénoalkyle.

9. Composé pour une utilisation selon l'une quelconque des revendications précédentes, dans lequel R9' est l'hydrogène.

10. Composé pour une utilisation selon l'une quelconque des revendications précédentes, dans lequel R20 est un aryle ou benzofuranyle.

11. Composé pour une utilisation selon l'une quelconque des revendications précédentes, dans lequel R6, R6', R7, R7', R8, R9, R9' et R10 sont des hydrogènes.

12. Composé pour une utilisation selon l'une quelconque des revendications précédentes, dans lequel R8' est un 1,1-diméthylpropyle ou trifluorométhyle.

13. Composé pour une utilisation selon l'une quelconque des revendications précédentes, dans lequel R21 est un hétéroaryle à 5 ou 6 chaînons éventuellement substitué.

14. Composé pour une utilisation selon l'une quelconque des revendications précédentes, dans lequel R21 est l'acide furane-2-carboxylique ou l'acide thiazol-4-ylacétique.

15. Composé selon l'une quelconque des revendications précédentes pour une utilisation selon l'une quelconque des revendications précédentes, dans lequel ladite maladie ou ledit trouble neurodégénératif associé à une fonction axonale compromise est une amyotrophie spinale.

16. Composé pour une utilisation selon l'une quelconque des revendications précédentes, lequel composé est au moins l'un choisi parmi les suivants :
acide 5-[5-(1,1-diméthylpropyl)-2-phényl-4,5,6,7-tétrahydroindol-1-yl]furane-2-carboxylique ;
acide 5-[2-(3,4-dichlorophényl)-5-trifluorométhyl-4,5,6,7-tétrahydroindol-1-yl]furane-2-carboxylique ;
acide {2-[5-(1,1-diméthylpropyl)-2-phényl-4,5,6,7-tétrahydroindol-1-yl]thiazol-4-yl}acétique ;
acide 6-(2-phényl-4,5,6,7-tétrahydro-1H-indol-1-yl)picolinique ;
acide 4-(2-phényl-4,5,6,7-tétrahydro-1H-indol-1-yl)picolinique ;
acide 5-(2-phényl-4,5,6,7-tétrahydro-1H-indol-1-yl)nicotinique ;
acide 2-(2-phényl-4,5,6,7-tétrahydro-1H-indol-1-yl)isonicotinique ;
acide 4-(2-phényl-4,5,6,7-tétrahydro-1H-indol-1-yl)nicotinique ;
acide 2-(2-phényl-4,5,6,7-tétrahydro-1H-indol-1-yl)pyrimidine-4-carboxylique ;
acide 5-(2-phényl-4,5,6,7-tétrahydro-1H-indol-1-yl)-1H-pyrrole-2-carboxylique ;
acide 4-(2-phényl-4,5,6,7-tétrahydro-1H-indol-1-yl)furane-2-carboxylique ;
ester méthylique d'acide 5-(5-tert-butyl-2-phényl-4,5,6,7-tétrahydroindol-1-yl)furane-2-carboxylique
acide 5-(5-tert-butyl-2-phényl-4,5,6,7-tétrahydroindol-1-yl)furane-2-carboxylique ;
acide 5-(2-phényl-5-trifluorométhyl-4,5,6,7-tétrahydroindol-1-yl)-furane-2-carboxylique ;
acide 5-[2-(4-morpholin-4-yl-phényl)-5-trifluorométhyl-4,5,6,7-tétrahydroindol-1-yl]furane-2-carboxylique ;
acide 5-(5-cyclohexyl-2-phényl-4,5,6,7-tétrahydroindol-1-yl)-furane-2-carboxylique ;
acide 5-(2-benzofuran-2-yl-6-trifluorométhyl-4,5,6,7-tétrahydroindol-1-yl)-furane-2-carboxylique ;
acide 5-(2-phényl-6-trifluorométhyl-4,5,6,7-tétrahydroindol-1-yl)-furane-2-carboxylique ;
acide 5-(2-benzofuran-2-yl-5-trifluorométhyl-4,5,6,7-tétrahydroindol-1-yl)-furane-2-carboxylique ;
acide 5-[2-benzofuran-2-yl-5-(1,1-diméthylpropyl)-4,5,6,7-tétrahydroindol-1-yl]furane-2-carboxylique ;
acide 5-(6-méthyl-2-phényl-4,5,6,7-tétrahydroindol-1-yl)furane-2-carboxylique ;
acide 5-[5-tert-butyl-2-(3,4-dichlorophényl)-4,5,6,7-tétrahydroindol-1-yl]furane-2-carboxylique ;
acide 5-[5-tert-butyl-2-(4-trifluorométhylphényl)-4,5,6,7-tétrahydroindol-1-yl]furane-2-carboxylique ;
acide 5-(5-méthyl-2-phényl-4,5,6,7-tétrahydroindol-1-yl)furane-2-carboxylique ;
acide 2-(5-méthyl-2-phényl-4,5,6,7-tétrahydroindol-1-yl)thiazole-4-carboxylique ;
acide 5-[2-(3,4-dichlorophényl)-4-trifluorométhyl-4,5,6,7-tétrahydroindol-1-yl]furane-2-carboxylique ;
acide 5-[2-(3,4-dichlorophényl)-6-trifluorométhyl-4,5,6,7-tétrahydroindol-1-yl]furane-2-carboxylique ;
5-tert-butyl-2-phényl-1-(4H-[1,2,4]triazol-3-yl)-4,5,6,7-tétrahydro-1H-indole ;
acide 2-[2-(2,4-diméthoxyphényl)-5-trifluorométhyl-4,5,6,7-tétrahydroindol-1-yl]thiazole-4-carboxylique ;
acide 2-[2-(3,4-dimethoxyphényl)-5-trifluorométhyl-4,5,6,7-tétrahydroindol-1-yl]thiazole-4-carboxylique ;
acide {2-[2-(3-chlorophényl)-5-(1,1-diméthylpropyl)-4,5,6,7-tétrahydroindol-1-yl]thiazol-4-yl}acétique ;
et les sels pharmaceutiquement acceptables de n'importe lesquels des précédents.
